(19) 

Europäisches
Patentamt
European
Patent Office
Office européen
des brevets

(11) **EP 4 353 267 A1**

(12) **EUROPEAN PATENT APPLICATION**
published in accordance with Art. 153(4) EPC

(43) Date of publication:
**17.04.2024 Bulletin 2024/16**

(21) Application number: **22804082.0**

(22) Date of filing: **20.05.2022**

(51) International Patent Classification (IPC):
**A61K 47/68** (2017.01)      **A61K 47/69** (2017.01)
**A61K 31/704** (2006.01)      **A61P 35/00** (2006.01)

(52) Cooperative Patent Classification (CPC):
**A61K 31/704; A61K 47/68; A61K 47/69;
A61P 35/00**

(86) International application number:
**PCT/CN2022/094222**

(87) International publication number:
**WO 2022/242763 (24.11.2022 Gazette 2022/47)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **21.05.2021   CN 202110560605**

(71) Applicant: **HighField BioPharmaceutical
Corporation
HangZhou, Zhejiang 310051 (CN)**

(72) Inventors:
 • **XU, Yuhong
   HangZhou, Zhejiang 310051 (CN)**

 • **JIN, Shanshan
   HangZhou, Zhejiang 310051 (CN)**
 • **CHEN, Xiaolong
   HangZhou, Zhejiang 310051 (CN)**

(74) Representative: **HGF
HGF Limited
1 City Walk
Leeds LS11 9DX (GB)**

Remarks:
The complete document including Reference
Table(s) and the Sequence Listing(s) can be
downloaded from the EPO website

(54) **ANTIBODY-CONJUGATED LIPOSOME**

(57)     An antibody-conjugated liposome, particularly a nanoparticle. The surface of the nanoparticle contains antibodies. The number of the antibodies is 5-60, preferably 5-50, and more preferably 5-40. The nanoparticle, for example, a liposome, can more effectively exert a therapeutic effect on tumor and improve multidrug resistance of tumor, thereby overcoming the technical prejudice that it is commonly considered that the more antibodies on a surface of a liposome, the better the target cell binding effect, and laying a foundation for further clinical development.

EP 4 353 267 A1

**Description**

**Technical Field**

The present disclosure belongs to the pharmaceutical field, and specifically relates to an antibody-conjugated liposome.

**Background Art**

There are multiple liposomal pharmaceutical products on the market at home and abroad, especially liposomal formulations containing antitumor chemotherapeutic drugs such as doxorubicin, irinotecan, and paclitaxel. On the one hand, encapsulation can alter the in vivo distribution and circulation characteristics of liposomal drugs, and on the other hand, the concentration of drugs accumulated in tumor tissues (passive targeting effect) can be increased through the EPR effect, and the therapeutic index can be significantly improved by improving the efficacy and/or reducing toxic side effects. However, due to the lack of specific interactions between traditional liposomes and tumor cells, drugs in liposomes need to be released from the liposomes before they can be taken up by tumor cells. They cannot be utilized, and hence drug efficacy cannot be fully maximized, thus limiting the application of liposomes.

Therefore, there is a need in this field to develop a liposome that is for the effective treatment of tumors.

**Summary of the Invention**

The object of the present invention is to provide a liposome for the effective treatment of tumors.

In the first aspect of the present disclosure, a nanoparticle is provided, and the surface of the nanoparticle contains antibodies.

The number of the antibodies is 5 - 60, preferably 5 - 50, and more preferably 5-40.

In another preferred example, the number of the antibodies is 5 - 30, preferably 5 - 25, preferably 5 - 20, more preferably 5 - 15, more preferably 8 - 12, and most preferably 10.

In another preferred example, the number of the antibodies is 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39 or 40.

In another preferred example, the outer surface of the nanoparticle contains antibodies.

In another preferred example, the antibodies are conjugated to the nanomaterial.

In another preferred example, the nanomaterial comprises a lipid material.

In another preferred example, the antibodies comprise HER2 antibodies (human epidermal growth factor receptor 2 antibodies).

In another preferred example, the HER2 antibodies comprise Fab fragments, Fab' fragments, F(ab')2 fragments, or single-chain Fv fragments (scFv) of HER2 antibodies.

In another preferred example, the Fab fragments of the HER2 antibodies comprise heavy and light chains;

the heavy chains are selected from the following groups:

(1) the amino acid sequence shown in SEQ ID NO.: 1;
(2) a group formed by one or more amino acid residue substitutions, deletions, or additions to the amino acid sequence shown in SEQ ID NO.:1;
(3) a group having 80%, preferably 90%, more preferably 95%, more preferably 98%, or more preferably 99% homology with the amino acid sequence shown in SEQ ID NO.: 1;≥≥≥≥≥

the light chains are selected from the following groups:

(a) the amino acid sequence shown in SEQ ID NO.: 2;
(b) a group formed by one or more amino acid residue substitutions, deletions, or additions to the amino acid sequence shown in SEQ ID NO.: 2;
(c) a group having ≥80%, preferably ≥90%, more preferably ≥95%, more preferably ≥98%, or more preferably ≥99% homology with the amino acid sequence shown in SEQ ID NO.: 2.

In another preferred example, the antibodies further comprise a second antibody, and the two antibodies are of different types.

In another preferred example, the second antibody comprises CD3 antibodies (human cluster of differentiation 3 antibodies).

In another preferred example, the CD3 antibodies comprise Fab fragments, Fab' fragments, F(ab')2 fragments, or single-chain Fv fragments (scFv) of CD3 antibodies.

In another preferred example, the number of the second antibody is 0 - 60, preferably 3 - 40, preferably 3 - 36, more preferably 6 - 18, and more preferably 9.

In another preferred example, the Fab fragments of the CD3 antibodies comprise heavy and light chains;

the heavy chains are selected from the following groups:

(1) the amino acid sequence shown in SEQ ID NO.: 3;
(2) a group formed by one or more amino acid residue substitutions, deletions, or additions to the amino acid sequence shown in SEQ ID NO.: 3;
(3) a group having ≥80%, preferably ≥90%, more preferably ≥95%, even more preferably ≥98%, or even more preferably ≥99% homology with the amino acid sequence shown in SEQ ID NO.: 3;

the light chains are selected from the following groups:

(a) the amino acid sequence shown in SEQ ID NO.: 4;
(b) a group formed by one or more amino acid residue substitutions, deletions, or additions to the amino acid sequence shown in SEQ ID NO.: 4;
(c) a group having ≥80%, preferably ≥90%, more preferably ≥95%, more preferably ≥98%, or more preferably ≥99% homology with the amino acid sequence shown in SEQ ID NO.: 4.

In another preferred example, the number of antibodies on the surface of the nanoparticle is calculated based on the following formula:

$$P = \frac{\frac{Y}{L} * NA}{N} * X * \frac{1}{NA} * MW = \frac{X * Y * MW}{N * L} \; ;$$

where X is the number of antibodies contained on the surface of each liposome;
MW is the average relative molecular mass of the protein;
Y is the lipid mass;
L is the average relative molecular mass of the lipids;
N is the average number of lipid molecules contained in each liposome; NA is the Avogadro constant;
P is the amount of antibodies required to prepare each liposome with X antibody molecules on the surface.

In another preferred example, the nanoparticle is a liposome or nanosized particle.
In another preferred example, the nanoparticle is a drug-loaded nanoparticle.
In another preferred example, the nanoparticle is a drug-loaded liposome.
In another preferred example, the nanoparticle is a drug-loaded liposome with an outer surface modified with antibodies.
In another preferred example, the nanosized particle is a drug-loaded nanosized particle.
In another preferred example, the loading involves the drug being encapsulated within the nanoparticle (e.g. a liposome or nanosized particle).
In another preferred example, the drug comprises antitumor drugs.
In another preferred example, the antitumor drugs are selected from the following group: anthracyclines, platinum-based drugs, fluorouracils, camptothecins, taxanes, or combinations thereof.
In another preferred example, the antitumor drugs comprise immunomodulatory drugs.
In another preferred example, the antitumor drugs comprise doxorubicin or resiquimod.
In another preferred example, the liposome comprises a lipid material.
In another preferred example, the lipid material is the lipid bilayer material of the liposome.
In another preferred example, the lipid material comprises one or more of hydrogenated soy phosphatidylcholine (HSPC), distearoyl phosphatidylethanolamine-polyethylene glycol (DSPE-mPEG), phosphatidylcholine, phosphatidylglycerol, phosphatidylserine, phosphatidylethanolamine, sphingomyelin, cholesterol, polyethylene glycol glycerol fatty acid esters, and polyethylene glycol glyceryl phosphatidylethanolamine.
In another preferred example, the antibodies are conjugated to the lipid material. In another preferred example, the antibodies are conjugated to DSPE-mPEG.

In another preferred example, the antibodies are conjugated to the liposome via DSPE-mPEG.

In another preferred example, the DSPE-mPEG comprises DSPE-mPEG2000.

In another preferred example, the free thiol group on the cysteine at position 229 of the heavy chains of the HER2 antibodies are conjugated to DSPE-mPEG.

In another preferred example, the method of conjugation comprises the following:

DSPE-PEG reacts with maleimide to form DSPE-PEG-MAL, and then the maleimide group of DSPE-PEG-MAL reacts with the free thiol group on the cysteine at position 229 of the heavy chains of the HER2 antibodies, resulting in the HER2 antibodies being conjugated to DSPE-PEG.

In another preferred example, the particle size of the nanoparticle is 60 - 250 nm.

In another preferred example, the particle size of the nanoparticle is 80 - 120 nm.

In another preferred example, the nanoparticle has a PDI of less than 0.1, more preferably less than 0.09, and more preferably less than 0.05.

In another preferred example, in the liposome, the weight ratio of the drug to the lipid material (drug-to-lipid ratio) is 0.02 - 0.1, preferably 0.022 - 0.085, more preferably 0.025 - 0.05, more preferably 0.028 - 0.04, or more preferably 0.033 - 0.037.

In another preferred example, the liposome is a drug-loaded liposome in which the weight ratio (drug-to-lipid ratio) of the drug to the lipid material is 1: 1-100, preferably 1: 10-70, preferably 1:25-35, or preferably 1:28-32.

In another preferred example, the antibodies in the nanoparticle are modified using a post-insertion method.

In another preferred example, the liposome is prepared by the following method:

(i) add the lipid material into an organic solvent and mix to obtain a lipid mixture;
(ii) add the lipid mixture into an aqueous phase to obtain an empty liposome solution.
(iii) Add the drug solution into the empty liposome solution and incubate to obtain the drug-loaded liposome;
(iv) mix the lipid material conjugated to the antibodies with the drug-loaded liposome mixture and incubate to obtain the liposome.

In another preferred example, the lipid material in step (i) is selected from the following group: HSPC, cholesterol (Chol), and DSPE-mPEG, or combinations thereof.

In another preferred example, the weight ratio of the HSPC to the Chol is 2-4:1.

In another preferred example, the weight ratio of the HSPC to DSPE-mPEG is 3-20:1.

In another preferred example, the weight ratio of the HSPC to DSPE-mPEG is 13-17:1.

In another preferred example, the organic solvent in step (ii) comprises ethanol.

In another preferred example, the aqueous phase in step (ii) is water or an ammonium sulfate aqueous solution.

In another preferred example, the concentration of the ammonium sulfate aqueous solution in step (ii) is 100 - 450 mM.

In another preferred example, the concentration of the ammonium sulfate aqueous solution in step (ii) is 150 - 270 mM.

In another preferred example, the pH of the ammonium sulfate aqueous solution in step (ii) is 4.0 - 6.0.

In another preferred example, the pH of the ammonium sulfate aqueous solution in step (ii) is 4.8 - 5.2.

In another preferred example, in step (ii), the lipid mixture is added into an aqueous phase, extruded with stirring, and then dialyzed to obtain an empty liposome solution.

In another preferred example, the dialysis is performed in HEPES buffer (8 - 12 mM, pH 6.6 - 6.8).

In another preferred example, the volume ratio of the organic solvent to the aqueous phase is 1:8-12.

In another preferred example, in step (iii), the weight ratio of the drug to the empty liposome is:

(drug-to-lipid ratio) is 0.02 - 0.1, preferably 0.022 - 0.085, more preferably 0.025 - 0.05, more preferably 0.028 - 0.04, or more preferably 0.033 - 0.037.

In another preferred example, in step (iii), the weight ratio of the drug to the empty liposome (drug-to-lipid ratio) is 1:25-35, preferably 1:28-32.

In the second aspect of the present disclosure, a composition is provided, and the composition comprises the nanoparticle according to the first aspect of the present disclosure.

In another preferred example, the composition is a pharmaceutical composition.

In another preferred example, the pharmaceutical composition further comprises a pharmaceutically acceptable carrier.

In another preferred example, the dosage form of the pharmaceutical composition is an oral formulation, an injectable formulation, or a topical formulation.

In another preferred example, the dosage form of the pharmaceutical composition is selected from the following: tablet, capsule, granule, suspension, pill, solution, syrup, or injection.

In another preferred example, the dosage form of the pharmaceutical composition is an injection.

In another preferred example, the injection is selected from the following: liquid formulation, suspension formulation, and lyophilized powder for injection.

In another preferred example, the injection is selected from the following: intravenous injection, subcutaneous injection, and in situ injection.

In the third aspect of the present disclosure, a use is provided for the nanoparticle according to the first aspect of the present disclosure or the composition according to the second aspect of the present disclosure, for the preparation of drugs used in the prevention and/or treatment of tumors.

In another preferred example, the tumors are selected from the following group: breast cancer, gastric cancer, ovarian cancer, liver cancer, or combinations thereof.

In another preferred example, the tumors comprise receptor-expressing tumors.

In another preferred example, the receptors comprise HER2.

In another preferred example, the receptors are conjugated to the antibodies.

In another preferred example, the conjugation comprises specific binding.

In another preferred example, the tumors comprise HER2-overexpressing tumors.

In another preferred example, HER2 overexpression means that compared to the HER2 expression of A0 in normal cells, the HER2 expression in tumor cells is A1, and A1/A0 > 1.2, preferably > 1.5.

In another preferred example, the tumors comprise tumors that have multidrug resistance to antitumor drugs.

In the fourth aspect of the present disclosure, a method is provided for the prevention and/or treatment of tumors by administering the nanoparticle according to the first aspect of the present disclosure or the composition according to the second aspect of the present disclosure to a subject in need.

In another preferred example, the subject comprises humans or non-human mammals.

It should be understood that within the scope of the present disclosure, the various technical features mentioned above and those specifically described in the following sections (such as in the embodiments) may be combined with each other to form new or preferred technical solutions. Due to space limitations, they will not be enumerated in detail here.

**Description of Attached Drawings**

Figure 1A shows the results of non-reducing SDS-PAGE detection of the connection between HER2Fab and DSPE-PEG2000-MAL.

Figure 1B shows the results of RP-HPLC detection of the connection between HER2Fab and DSPE-PEG2000-MAL.

Figure 1C shows the results of non-reducing SDS-PAGE detection of the purification of aHER2-DSPE.

Figure 1D shows the results of RP-HPLC detection of the purification of aHER2-DSPE.

Figure 2 shows the particle size distribution of doxorubicin liposomes (aHER2-LDX) inserted with different numbers of antibodies, where Figures 2A, 2B and 2C show the particle size distribution of aHER2-LDX with an antibody count of 10, 20 and 40, respectively.

Figure 3 shows the post-insertion efficiency of aHER2-DSPE after insertion into LDX as detected by a Sepharose 4B gel column.

Figure 4 illustrates the flow cytometry-based detection of HER2 antigen expression on the surfaces of SK-Br-3, BT474, NCI-N87, MDA-MB-453, MCF-7, MDA-MB-231, MGC-803, and A2780 cells, where rMFI indicates the relative mean fluorescence intensity.

Figure 5A shows the inhibition of tumor cell growth in the HER2-overexpressing cell line SK-Br-3 by 10-C aHER2-LDX, 20-C aHER2-LDX, 10-B aHER2-LDX, 20-B aHER2-LDX, and A-LDX.

Figure 5B shows the inhibition of tumor cell growth in the HER2-overexpressing cell line NCI-N87 by 10-C aHER2-LDX, 20-C aHER2-LDX, 10-B aHER2-LDX, 20-B aHER2-LDX, and A-LDX.

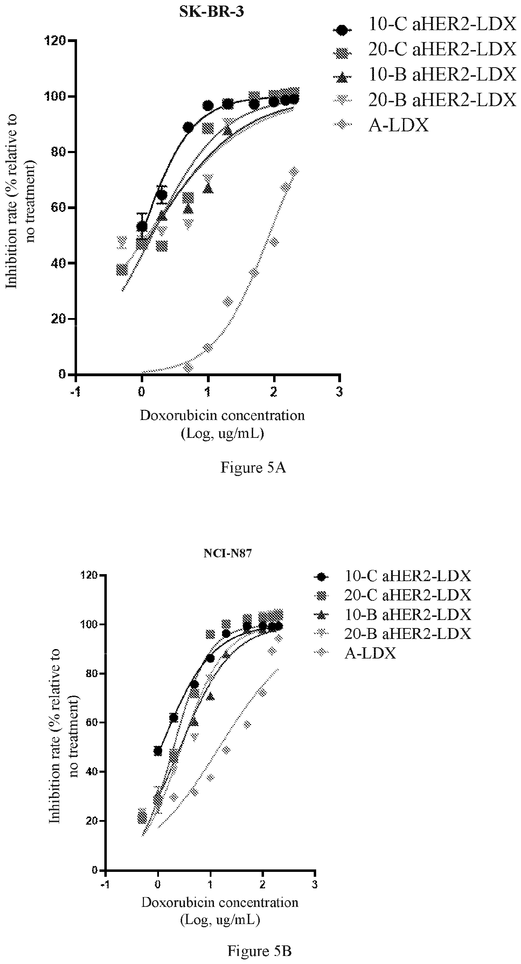

Figure 5A shows the inhibition of tumor cell growth in the HER2-overexpressing cell line BT474 by 10-C aHER2-LDX, 20-C aHER2-LDX, 10-B aHER2-LDX, 20-B aHER2-LDX, and A-LDX.

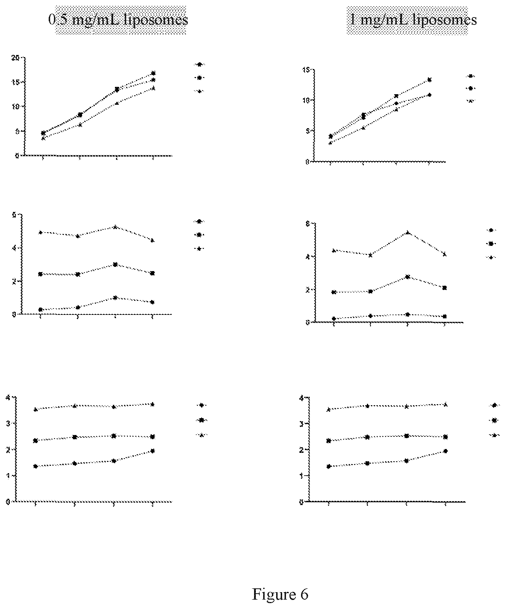

Figure 6 shows the affinity activity of HER2 antibody-immunoliposomes as detected by the Gator label-free bioanalyzer. On the left, the lipid concentration is 0.5 mg/mL, and on the right, the lipid concentration is 1 mg/mL.

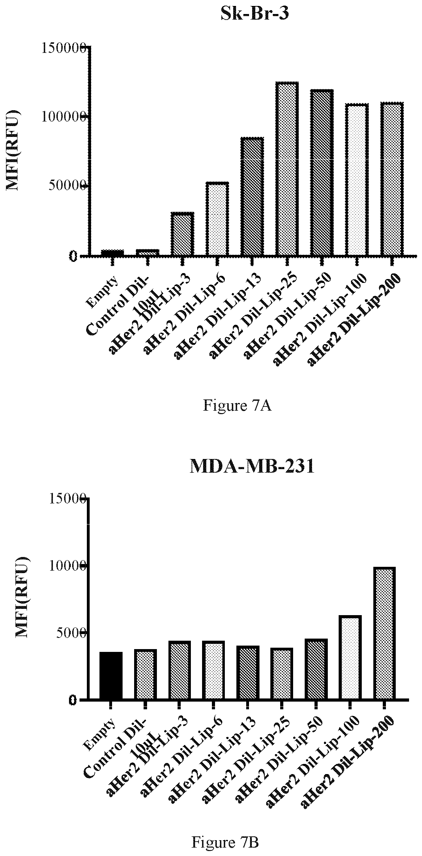

Figure 7A shows the in vitro binding intensity of the HER2Fab immunofluorescent liposome aHER2Dil-Lip to the HER2-overexpressing cell line SK-Br-3 at different antibody densities, where aHER2Dil-Lip-3 represents fluorescently labeled aHER2-LDX conjugated to three HER2Fab antibody fragments, and so on.

Figure 7B shows the in vitro binding intensity of the fluorescent liposome aHER2Dil-Lip of HER2Fab at different antibody densities to the HER2-negative cell line MDA-MB-231, where aHER2Dil-Lip-3 represents fluorescently labeled aHER2-LDX conjugated to three HER2Fab antibody fragments, and so on.

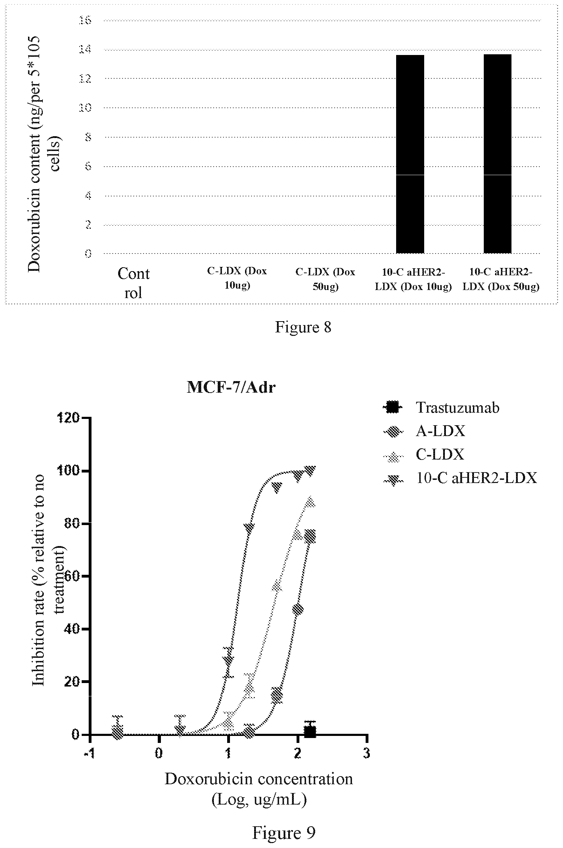

Figure 8 shows the uptake and drug release of HER2 antibody-conjugated doxorubicin-loaded liposomes by the HER2-overexpressing cell line BT474.

Figure 9 shows the cytotoxic effect of HER2Fab antibody-targeted doxorubicin-loaded liposomes on doxorubicin-resistant breast cancer cells MCF-7/Adr.

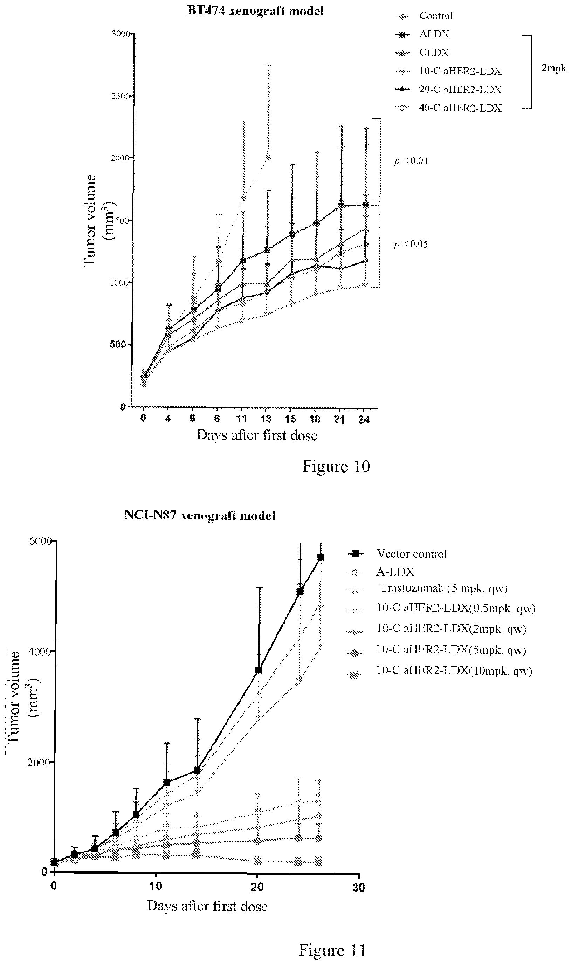

Figure 10 shows the pharmacodynamics of aHER2-LDX in a xenograft model of HER2-overexpressing BT474 breast cancer in Balb/C-Nude mice.

Figure 11 shows the pharmacodynamics of aHER2-LDX in a xenograft model of HER2-overexpressing NCI-N87 gastric cancer in Balb/C-Nude mice.

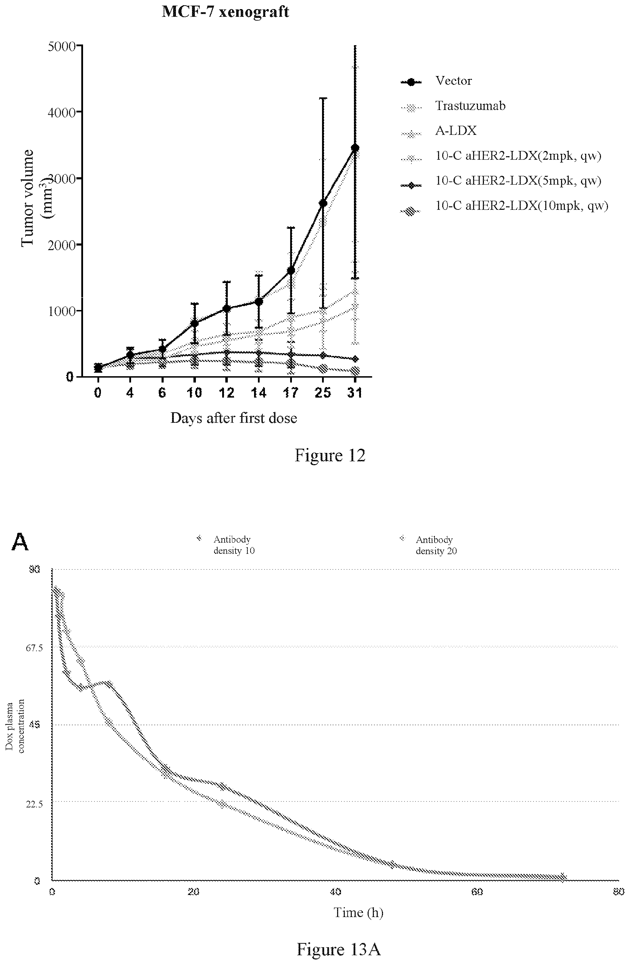

Figure 12 shows the pharmacodynamics of aHER2-LDX in a xenograft model of HER2-low expressing MCF-7 breast cancer in Balb/C-Nude mice.

Figure 13A shows the pharmacokinetics of aHER2-LDX in BT474 tumor-bearing mice for the doxorubicin drug.

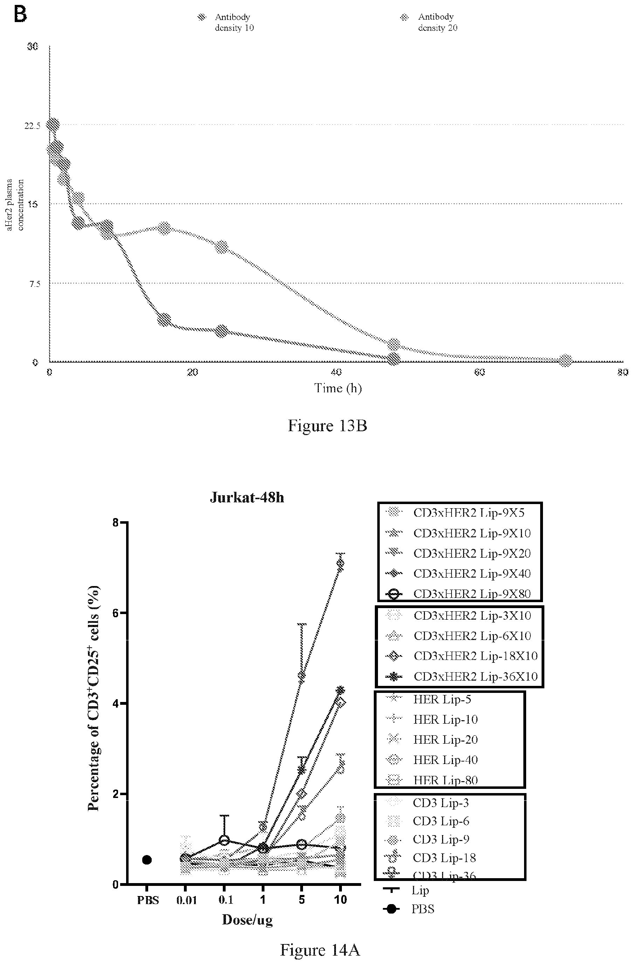

Figure 13B shows the pharmacokinetics of aHER2-LDX in BT474 tumor-bearing mice for HER2Fab.

Figure 14A shows the in vitro activation of CD25 expression on the surface of Jurkat cells by CD3 × HER2 dual-targeting liposomes at different densities.

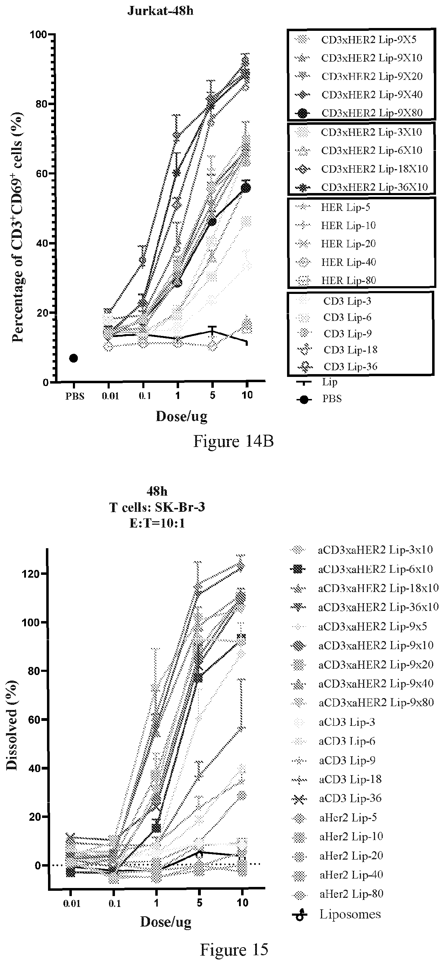

Figure 14B shows the in vitro activation of CD69 expression on the surface of Jurkat cells by CD3 × HER2 dual-targeting liposomes at different densities.

Figure 15 shows the cytotoxicity of pre-activated effector cells (PBMCs) mediated by CD3 × HER2 dual-targeting liposomes against the target cells SK-Br-3 at different densities, detected by LDH assay after 48 hours of drug exposure.

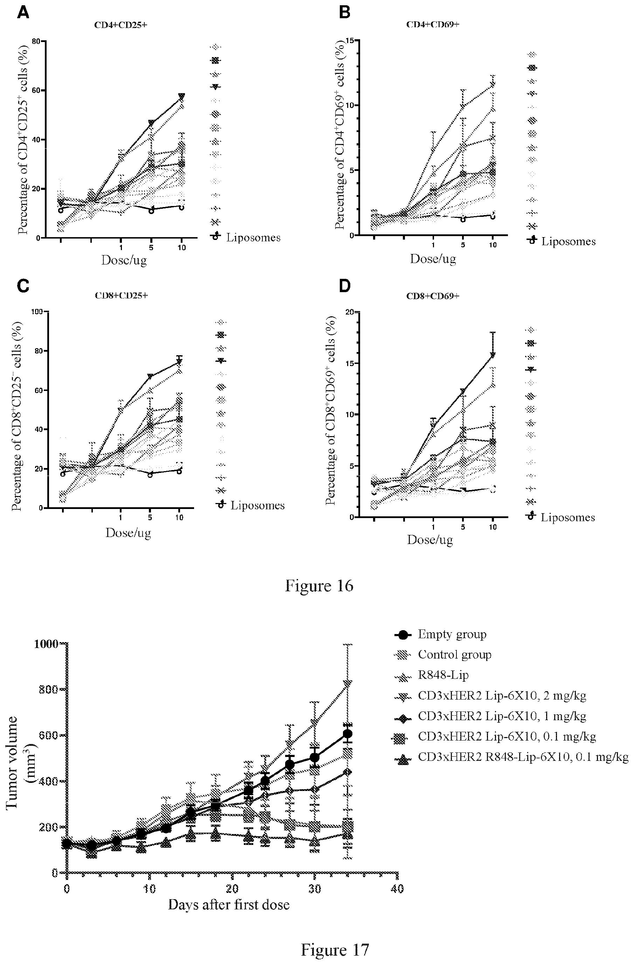

Figure 16 shows the expression of CD25 and CD69 on the surface of T cells after 48 hours of drug exposure, detected by flow cytometry, in co-cultures of pre-activated effector cells (PBMCs) with the target cells SK-Br-3 at different densities mediated with CD3 × HER2 dual-targeting liposomes. (A) CD25 expression in CD4-positive cells; (B) CD69 expression in CD4-positive cells; (C) CD25 expression in CD8-positive cells; (D) CD69 expression in CD8-positive cells.

Figure 17 shows the pharmacodynamics of CD3 × HER2 dual-targeting liposomes and CD3 × HER2 dual-targeting R848-loaded liposomes in a humanized model of HER2-overerepressing NCI-N87 gastric cancer in NCG-B2M-KO severely immunodeficient mice.

## Specific Embodiments

After extensive and in-depth research, the inventors have developed a nanoparticle, such as liposome, and the surface of the nanoparticle contains antibodies. Studies on the embodiments have shown that liposome surfaces containing 10 - 40 liposomes can more effectively exert a therapeutic effect on tumors and reduce multidrug resistance in tumors. The present invention is completed on this basis.

## Terms

As used herein, "pharmaceutical composition of the present disclosure" refers to a pharmaceutical composition comprising an antitumor drug and a liposome, and the composition has a drug-to-lipid ratio in the range of 0.01 - 0.15, preferably 0.02 - 0.1.

As used herein, human epidermal growth factor receptor 2, abbreviated as HER2, aHER2, or [sic] HER2, is also referred to as Neu or HER2/neu.

As used herein, the representative names of individual cell lines are shown in Table A below:

Table A Cell Lines

| Cell Line | Description |
|---|---|
| Sk-Br-3 | Human breast cancer cell line |
| BT474 | Human breast cancer cell line |
| NCI-N87 | Human gastric cancer cell line |
| MDA-MB-231 | Human triple-negative breast cancer cell line |
| MDA-MB-453 | Human breast cancer cell line |
| MCF-7 | Human breast cancer cell line |
| A2780 | Human ovarian cancer cell line |
| MGC-803 | Human gastric cancer cell line |

**Antibodies**

In a preferred example of the present disclosure, the antibodies described herein comprise HER2 antibodies (human epidermal growth factor receptor 2 antibodies).

The basic unit structure of a complete antibody consists of four peptide chains, including two heavy and two light chains, bound together by disulfide bonds. The shape of an antibody resembles the letter Y, and the hinge region of the Y structure is flexible. Each peptide chain has a constant region (highly conserved in all antibodies) and a variable region (specific to a particular antibody). The light chain variable region is labeled VL, while the light chain constant region is labeled CL. Similarly, the variable and constant regions of the heavy chains are labeled as VH and CH, respectively. Carbohydrates typically bind to the CH2 region of the heavy chain. The Fc region comprises only the constant region (CH) of the heavy chains, while the antigen-binding Fab region (Fab) comprises the variable regions of the heavy and light chains, as well as a constant region connected to the variable regions of both the heavy and light chains.

In a preferred example of the present disclosure, the HER2 antibodies comprise Fab fragments, Fab' fragments, F(ab')2 fragments, or single-chain Fv fragments (scFv) of HER2 antibodies.

Representatively, the Fab fragments of the HER2 antibodies comprise heavy and light chains;

the heavy chains are selected from the following groups:

(1) the amino acid sequence shown in SEQ ID NO.: 1;
(2) a group formed by one or more amino acid residue substitutions, deletions, or additions to the amino acid sequence shown in SEQ ID NO.: 1;
(3) a group having ≥80%, preferably ≥90%, more preferably ≥95%, more preferably ≥98%, or more preferably ≥99% homology with the amino acid sequence shown in SEQ ID NO.: 1;

the light chains are selected from the following groups:

(a) the amino acid sequence shown in SEQ ID NO.: 2;
(b) a group formed by one or more amino acid residue substitutions, deletions, or additions to the amino acid sequence shown in SEQ ID NO.: 2;
(c) a group having ≥80%, preferably ≥90%, more preferably ≥95%, more preferably ≥98%, or more preferably ≥99% homology with the amino acid sequence shown in SEQ ID NO.: 2.

**Nanoparticle**

The present disclosure provides a nanoparticle, and the surface of the nanoparticle contains antibodies;

The number of the antibodies is 5 - 60, preferably 5 - 50, and more preferably 5-40.

In a preferred example of the present disclosure, the number of the antibodies is 5 - 30, preferably 5 - 25, preferably 5 - 20, more preferably 5 -15, more preferably 8 - 12, and most preferably 10.

Representatively, the number of the antibodies is 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39 or 40.

In a preferred example of the present disclosure, the outer surface of the nanoparticle contains antibodies.

In a preferred example of the present disclosure, the antibodies are conjugated to the nanomaterial.

In a preferred example of the present disclosure, the nanomaterial comprises a lipid material.

In a preferred example of the present disclosure, the nanoparticle is a liposome or nanosized particle.

In another preferred example, the nanoparticle is a drug-loaded nanoparticle.

In another preferred example, the nanoparticle is a drug-loaded liposome.

In another preferred example, the nanosized particle is a drug-loaded nanosized particle.

In a preferred example of the present disclosure, the loading involves the drug being encapsulated within the nanoparticle (e.g. a liposome or nanosized particle).

In a preferred example of the present disclosure, the drug comprises antitumor drugs.

Representatively, the antitumor drugs are selected from the following group: anthracyclines, platinum-based drugs, fluorouracils, camptothecins, taxanes, or combinations thereof.

Representatively, the antitumor drugs comprise doxorubicin.

In a preferred example of the present disclosure, the liposome comprises a lipid material.

In a preferred example of the present disclosure, the lipid material is the lipid bilayer material of the liposome.

Representatively, the lipid material comprises one or more of HSPC, DSPE-mPEG2000, phosphatidylcholine, phosphatidylglycerol, phosphatidylserine, phosphatidylethanolamine, sphingomyelin, cholesterol, polyethylene glycol glycerol fatty acid esters, and polyethylene glycol glyceryl phosphatidylethanolamine.

In a preferred example of the present disclosure, the antibodies are conjugated to the lipid material.

Representatively, the antibodies are conjugated to DSPE-PEG.

Representatively, the free thiol group on the cysteine at position 229 of the heavy chains of the HER2 antibodies is conjugated to DSPE-PEG.

In a preferred example of the present disclosure, the method of conjugation comprises the following: DSPE-PEG reacts with maleimide to form DSPE-PEG-MAL, and then the maleimide group of DSPE-PEG-MAL reacts with the free thiol group on the cysteine at position 229 of the heavy chains of the HER2 antibodies, resulting in the HER2 antibodies being conjugated to DSPE-PEG.

In another preferred example, the particle size of the nanoparticle is 60 - 250 nm.

In another preferred example, the particle size of the nanoparticle is 80 - 100 nm.

In a preferred example of the present disclosure, in the liposome, the weight ratio of the drug to the lipid material (drug-to-lipid ratio) is 0.02 - 0.1, preferably 0.022 - 0.085, more preferably 0.025 - 0.05, more preferably 0.028 - 0.04, or more preferably 0.033 - 0.037.

In a preferred example of the present disclosure, in the liposome, the weight ratio of the drug to the lipid material (drug-to-lipid ratio) is 1:25-35, preferably 1:28-32.

In a preferred example of the present disclosure, the liposome is prepared by the following method:

(i) add the lipid material into an organic solvent and mix to obtain a lipid mixture;

(ii) add the lipid mixture into an aqueous phase to obtain an empty liposome solution.

(iii) Add the drug solution into the empty liposome solution and incubate to obtain the drug-loaded liposome;

(iv) mix the lipid material conjugated to the antibodies with the drug-loaded liposome mixture and incubate to obtain the liposome.

In another preferred example, the lipid material in step (i) is selected from the following group: HSPC, Chol, and DSPE-mPEG2000, or combinations thereof.

In another preferred example, the weight ratio of the HSPC to the Chol is 2-4:1.

In another preferred example, the weight ratio of the HSPC to DSPE-mPEG2000 is 3-20:1.

In another preferred example, the weight ratio of the HSPC to DSPE-mPEG2000 is 15-17:1.

In another preferred example, the organic solvent in step (ii) comprises ethanol.

In another preferred example, the aqueous phase in step (ii) is water or an ammonium sulfate aqueous solution.

In another preferred example, the concentration of the ammonium sulfate aqueous solution in step (ii) is 100 - 450 mM.

In another preferred example, the concentration of the ammonium sulfate aqueous solution in step (ii) is 230 - 270 mM.

In another preferred example, the pH of the ammonium sulfate aqueous solution in step (ii) is 4.0 - 6.0.

In another preferred example, the pH of the ammonium sulfate aqueous solution in step (ii) is 4.8 - 5.2.

In another preferred example, in step (ii), the lipid mixture is added into an aqueous phase, extruded with stirring, and then dialyzed to obtain an empty liposome solution.

In another preferred example, the dialysis is performed in HEPES buffer (8 - 12 mM, pH 6.6 - 6.8).

In another preferred example, the volume ratio of the organic solvent to the aqueous phase is 1:8-12.

In another preferred example, in step (iii), the weight ratio of the drug to the empty liposome (drug-to-lipid ratio) is 0.02 - 0.1, preferably 0.022 - 0.085, more preferably 0.025 - 0.05, more preferably 0.028 - 0.04, or more preferably 0.033 - 0.037.

In another preferred example, in step (iii), the weight ratio of the drug to the empty liposome (drug-to-lipid ratio) is 1:25-35, preferably 1:28-32.

**Composition**

The present disclosure further provides a composition, and the composition comprises the nanoparticle according to the present disclosure.

In a preferred example of the present disclosure, the composition is a pharmaceutical composition.

In a preferred example of the present disclosure, the pharmaceutical composition further comprises a pharmaceutically acceptable carrier. As used herein, the term "pharmaceutically acceptable" ingredient is a substance that is suitable for use in humans and/or mammals without excessive adverse side effects (e.g., toxicity, irritation, and allergic reactions), i.e., a substance that has a reasonable benefit/risk ratio. The term "pharmaceutically acceptable carrier" refers to a carrier for the administration of a therapeutic agent, including various excipients and diluents. Such pharmaceutically acceptable carriers comprise (but are not limited to): saline, buffer, glucose, water and combinations thereof. Generally, the pharmaceutical formulation should match the mode of administration. The dosage form of the pharmaceutical composition of the present disclosure is selected from the following: tablet, capsule, granule, suspension, pill, solution, syrup, or injection.

In another preferred example, the dosage form of the pharmaceutical composition is an injection.

In another preferred example, the injection is selected from the following: injection, suspension, lyophilized powder for injection.

In another preferred example, the injection is selected from the following: intravenous injection, subcutaneous injection, and in situ injection.

Preparation is carried out by conventional methods with saline or an aqueous solution containing glucose and other adjuvants. The pharmaceutical composition is preferably manufactured under sterile conditions.

The effective amount of the active ingredient of the present disclosure may vary with the mode of administration and the severity of the disease to be treated. The selection of the preferred effective amount may be determined by one of ordinary skill in the art based on various factors (e.g., by clinical trials). Such factors include, but are not limited to the pharmacokinetic parameters of the active ingredient, such as bioavailability, metabolism, half-life, etc.; the severity of the patient's disease to be treated, the patient's weight, the patient's immune status, the route of administration, etc. Generally, satisfactory results can be obtained when the active ingredient of the present disclosure is administered at a dose of about 0.00001 mg -50 mg/kg of animal body weight per day (preferably 0.0001 mg - 10 mg/kg of animal body weight). For example, several separate doses may be given each day, or the dose may be proportionately reduced, as required by the exigencies of the treatment situation.

Typically, when the pharmaceutical composition of the present disclosure is administered orally, the average daily dose for a 60 kg individual (human) 10 - 500 mg, preferably 20 - 300 mg, and more preferably 50 - 250 mg in a 60 kg body weight subject (human). The daily dose may be taken in one, two, or more doses.

The pharmaceutically acceptable carriers according to the present disclosure comprise (but are not limited to) water, saline, nanogels, or combinations thereof. The choice of carriers should be compatible with the mode of administration, and this is well known to those of ordinary skill in the art.

In a preferred example of the present disclosure, the dosage form of the pharmaceutical composition is an oral formulation, an injectable formulation, or a topical formulation.

In a preferred example of the present disclosure, the dosage form of the pharmaceutical composition is selected from the following: tablet, capsule, granule, suspension, pill, solution, syrup, or injection.

In a preferred example of the present disclosure, the dosage form of the pharmaceutical composition is an injection.

In a preferred example of the present disclosure, the injection is selected from the following: liquid formulation, suspension formulation, and lyophilized powder for injection.

In a preferred example of the present disclosure, the mode of administration of the injection is selected from the following: intravenous injection, subcutaneous injection, and in situ injection.

**Tumors**

Tumors according to the present disclosure are preferably selected from the following group: breast cancer, gastric cancer, ovarian cancer, liver cancer, or combinations thereof.

In a preferred example of the present disclosure, the tumors comprise receptor-expressing tumors.

In another preferred example, the receptors comprise HER2.

In another preferred example, the receptors are conjugated to the antibodies.

In another preferred example, the conjugation comprises specific binding.

In a preferred example of the present disclosure, the tumors comprise HER2-overexpressing tumors.

In a preferred example of the present disclosure, HER2 overexpression means that compared to the HER2 expression of A0 in normal cells, the HER2 expression in tumor cells is A1, and A1/A0 > 1.2, preferably > 1.5.

In a preferred example of the present disclosure, the tumors comprise tumors that have multidrug resistance to antitumor drugs.

**Use and Methods**

The present disclosure further provides a use for the nanoparticle or the composition according to the present disclosure, for the preparation of drugs used in the prevention and/or treatment of tumors.

The present disclosure further provides a method for the prevention and/or treatment of tumors by administering the nanoparticle or the composition according to the present disclosure to a subject in need.

In a preferred example of the present disclosure, the subject comprises humans or non-human mammals.

**Key Advantages of the Present Disclosure Include the Following:**

In the present disclosure a nanoparticle, such as a liposome, is developed. When the surface of the nanoparticle, such as a liposome, contains 10 - 40 liposomes, it can more effectively exert a therapeutic effect on tumors and reduce the multidrug resistance of tumors. This overcomes the generally accepted technical bias that more antibodies on the surface of a liposome lead to better binding with target cells, thereby laying the foundation for further clinical development.

The present disclosure is further described below with specific embodiments. It should be understood that these embodiments are provided solely for the purpose of illustrating the present disclosure and are not intended to limit the scope of the present disclosure. In the following embodiments, experimental methods without specified conditions are typically conducted under conventional conditions or as recommended by the manufacturer. Unless otherwise stated, percentages and weight fractions are weight percentages and parts by weight.

**Embodiments**

The heavy chain amino acid sequence of the Fab fragment (aHER2 Fab) of HER2 antibodies is shown in SEQ ID NO.: 1, wherein the cysteine (C) at position 229 of the heavy chain contains a free thiol group connected to a linker (DSPE-PEG-MAL); the light chain amino acid sequence is shown in SEQ ID NO.: 2,

SEQ ID NO.: 1 (N-terminus to C-terminus):

EVQLVESGGGLVQPGGSLRLSCAASGFNIKDTYIHWVRQAPGKGLEWVARI

YPTNGYTRYADSVKGRFTISADTSKNTAYLQMNSLRAEDTAVYYCSRWGGDGFYA

MDYWGQGTLVTVSSASTKGPSVFPLAPSSKSTSGGTAALGCLVKDYFPEPVTVSW

NSGALTSGVHTFPAVLQSSGLYSLSSVVTVPSSSLGTQTYICNVNHKPSNTKVDKK

VEPKSCDKTHTCAA

SEQ ID NO.: 2 (N-terminus to C-terminus):

DIQMTQSPSSLSASVGDRVTITCRASQDVNTAVAWYQQKPGKAPKLLIYSAS

FLYSGVPSRFSGSRSGTDFTLTISSLQPEDFATYYCQQHYTTPPTFGQGTKVEIKRT

VAAPSVFIFPPSDEQLKSGTASVVCLLNNFYPREAKVQWKVDNALQSGNSQESVT

EQDSKDSTYSLSSTLTLSKADYEKHKVYACEVTHQGLSSPVTKSFNRGEC

The heavy chain amino acid sequence of the Fab fragment (aCD3 Fab) of CD3 antibodies is shown in SEQ ID NO.: 3, wherein the C at position 231 of the heavy chain contains a free thiol group connected to a linker (DSPE-PEG-MAL); the light chain amino acid sequence is shown in SEQ ID NO.: 4,

SEQ ID NO.: 3 (N-terminus to C-terminus):

EVQLVESGGGLVQPGGSLRLSCAASGYSFTGYTMNWVRQAPGKGLEWVA

LINPYKGVTTYADSVKGRFTISVDKSKNTAYLQMNSLRAEDTAVYYCARSGYYGDS

DWYFDVWGQGTLVTVSSASTKGPSVFPLAPSSKSTSGGTAALGCLVKDYFPEPVT

VSWNSGALTSGVHTFPAVLQSSGLYSLSSVVTVPSSSLGTQTYICNVNHKPSNTKV

DKKVEPKSCDKTHTCAA

SEQ ID NO.: 4 (N-terminus to C-terminus):

DIQMTQSPSSLSASVGDRVTITCRASQDIRNYLNWYQQKPGKAPKLLIYYTS
RLESGVPSRFSGSGSGTDYTLTISSLQPEDFATYYCQQGNTLPWTFGQGTKVEIKR
TVAAPSVFIFPPSDEQLKSGTASVVCLLNNFYPREAKVQWKVDNALQSGNSQESVT
EQDSKDSTYSLSSTLTLSKADYEKHKVYACEVTHQGLSSPVTKSFNRGEC

## 1. Preparation of Liposomes Encapsulating Doxorubicin (LDX)

900 mg of HSPC, 300 mg of Chol, and 60 mg of DSPE-mPEG2000 were added to 2 mL of anhydrous ethanol and heated in a water bath to 60°C. Then, a magnetic stirrer was added for stirring until complete dissolution, resulting in a clear liquid mixture, which was the lipid mixture. At the same time, 3.304 g of ammonium sulfate was placed in a glass bottle, added with 100 mL of ultrapure water, and stirred until dissolution, and the pH was then adjusted to 5.0 to obtain an ammonium sulfate aqueous solution with a concentration of 250 mM; this solution was prepared just before use. The dissolved lipid mixture was injected into 19 mL of the ammonium sulfate aqueous solution using a syringe, then stirred and extruded six times, and dialysis was then performed using a 10 kD dialysis membrane in a HEPES buffer (10 mM, pH 6.8) to obtain an empty liposome with an external aqueous phase of a HEPES solution.

10 mg/mL of doxorubicin stock solution was added to the corresponding doxorubicin stock solution and the above empty liposome suspension according to the drug and empty liposome concentrations set forth in Table 1, then incubated with stirring at 60°C for 30 min. After drug loading was completed, a 100kD dialysis membrane was used to remove the free drug and prepare Formulations A, B, C, and D of doxorubicin-loaded liposomes, denoted as: A-LDX, B-LDX, C-LDX, and D-LDX.

The Nano-S90 nanoparticle size analyzer was used for detection and the particle size of doxorubicin-loaded liposomes for different formulations was about 85 nm; the polydispersity index (PDI) was less than 0.1. Electron microscopy examination revealed that Formulations A, B, C, and D of doxorubicin-loaded liposomes were all round in shape with uniform size distribution.

Table 1

| Doxorubi cin-Loaded Liposome | Formulat ion | Doxorubicin Concentration (mg/mL) | Empty Liposome Concentration (mg/mL) |
|---|---|---|---|
| A-LDX | Formulat ion A | 2 | 15 |
| B-LDX | Formulat ion B | 1 | 15 |
| C-LDX | Formulat ion C | 1 | 30 |
| D-LDX | Formulat ion D | 0.5 | 30 |

## 2. Preparation of Immunoliposomes Modified with HER2 Fab Antibody Fragments (aHER2-LDX)

### 2.1 Preparation of HER2 Fab Antibody Fragment-Liposome Molecule (aHER2-DSPE)

The C of the HER2 Fab antibody at position 229 of the heavy chain contains a free thiol group, and this free thiol group (-SH) is exposed by reduction at a specific site. The free thiol group chemically reacts with the maleimide group of the lipid molecule DSPE-PEG-MAL to give the HER2 Fab antibody fragment-lipid molecule (denoted as follows: aHER2-DSPE). The specific procedure is as follows:

First, the concentration of the HER2 Fab antibody was determined by ultraviolet spectrophotometry or the BCA method. Reduction was then performed using the β-mercaptoethylamine (β-MEA) reducing agent. The HER2 Fab antibody and β-MEA were mixed in a molar ratio of 10:1, and the HER2 Fab antibody was reduced at room temperature for approximately 45 to 90 min. After reduction, desalting was performed using a Zeb desalting column or an AKTA instrument to remove the reducing agent from the solution. DSPE-PEG-MAL and DSPE-mPEG2000 were mixed in a molar ratio of 1:1 and dissolved in a 30 mM HEPES solution or PBS solution (containing 2 mM EDTA), with the pH adjusted to 6.5 -6.8, resulting in a mixed micelle solution of DSPE-PEG2-MAL and DSPE-mPEG2000. Subsequently, the HER2 Fab antibody and DSPE-PEG-MAL were separately added in a molar ratio of 1:1. The reaction was carried out at 4°C for 2 hours to give aHER2-DSPE micelles. Next, L-cysteine with a final concentration of 1 mM was added to block any unreacted DSPE-PEG-MAL, and the mixture was incubated at 4°C for less than 16 hours. As the reaction between the HER2 Fab antibody and DSPE-PEG-MAL was not complete, the resulting mixture consisted of the HER2 Fab antibody, DSPE-PEG-MAL, DSPE-mPEG2000, and the conjugate aHER2-DSPE. Therefore, purification was next performed using a gel column and ion-exchange column to obtain aHER2-DSPE single micelles.

The conjugate aHER2-DSPE was identified through non-reducing SDS-PAGE and RP-HPLC.

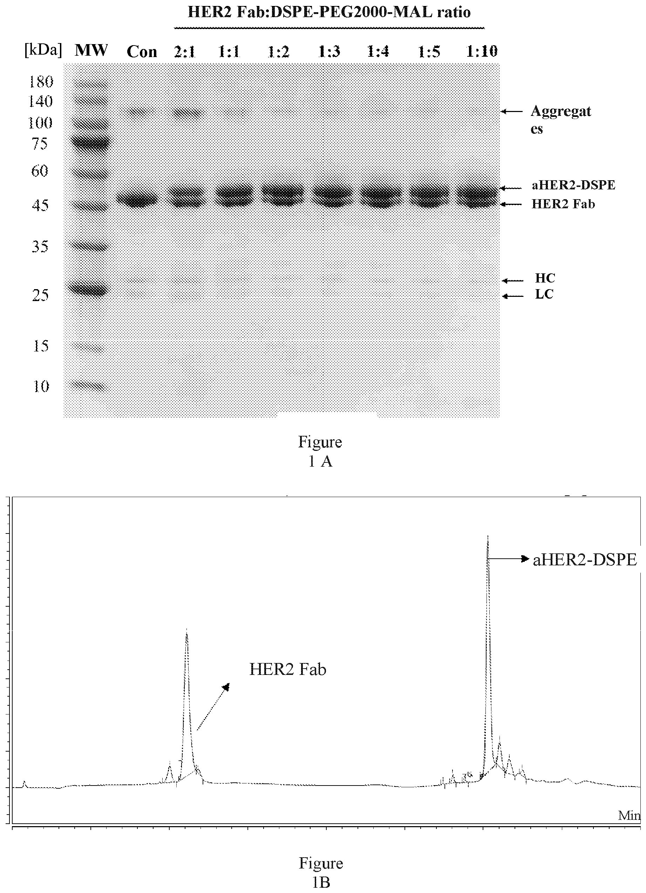

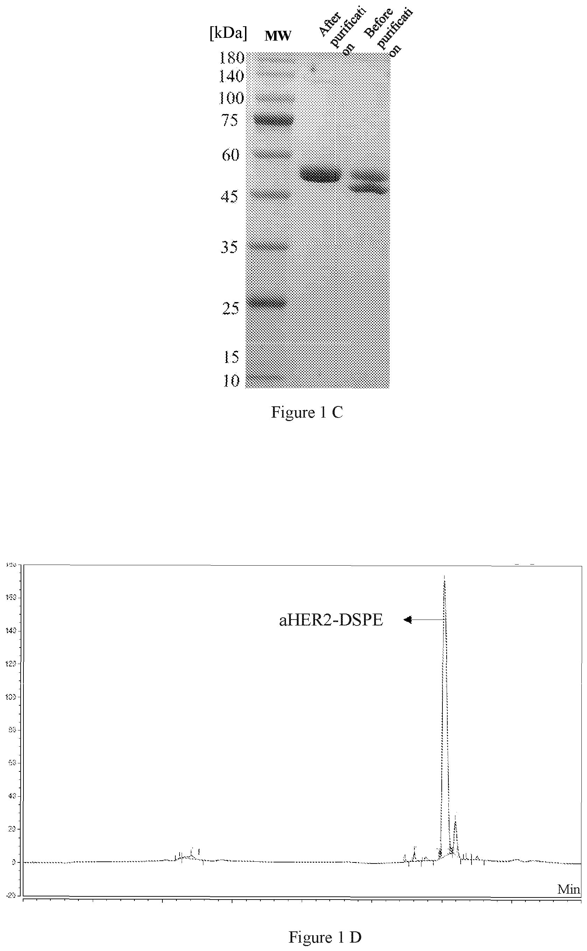

The results of Figure 1A from non-reducing SDS-PAGE gel analysis show that a molar ratio of 1:1 for the HER2 Fab antibody and DSPE-PEG-MAL is the preferred reaction ratio. In Figure 1B, the results indicate that, as detected by RP-HPLC, the conjugation efficiency is greater than 40% when the molar ratio of the HER2 Fab antibody to DSPE-PEG-MAL is 1:1. In Figures 1C and 1D, the purified aHER2-DSPE is identified using non-reducing SDS-PAGE and RP-HPLC methods, demonstrating a purity exceeding 95%.

**2. Preparation of Doxorubicin-Loaded Liposomes Modified with HER2 Fab Antibody Fragments (aHER2-LDX)**

In order to precisely prepare docetaxel-loaded liposomes with a specific number of HER2 Fab antibodies, a calculation method is provided, as detailed below:
Calculating based on the surface of one liposome being conjugated with X antibody molecules:
Known parameters:

(1) Average relative molecular mass of protein, denoted as MW;
(2) Lipid mass, denoted as Y;
(3) Average relative molecular mass of lipids, denoted as L (to be calculated based on lipid ratio);
(4) On average, each liposome contains N lipid molecules (to be calculated based on the average liposome size);
(5) Avogadro constant NA

The amount of lipid is Y, and to prepare each liposome with X antibody molecules on the surface, the required amount of antibodies, denoted as P, is:

Calculation formula:

$$P = \frac{\frac{Y}{L} * NA}{N} * X * \frac{1}{NA} * MW = \frac{X * Y*MW}{N * L}$$

aHER2-DSPE was anchored to the surface of doxorubicin-loaded liposomes (LDX) through a post-insertion method to prepare doxorubicin-loaded liposomes modified with the HER2 Fab antibody (aHER2-LDX). The specific procedure is as follows:
First, the antibody concentration of the prepared aHER2-DSPE and the lipid concentration of the doxorubicin-loaded liposome were separately determined. Following the experimental requirements, the amounts of aHER2-DSPE and doxorubicin-loaded liposome were calculated according to the above formula. The two were mixed well and incubated at 60°C for 30 min. After the reaction was completed, the mixture was allowed to return to room temperature and was then stored at 2 - 8°C.

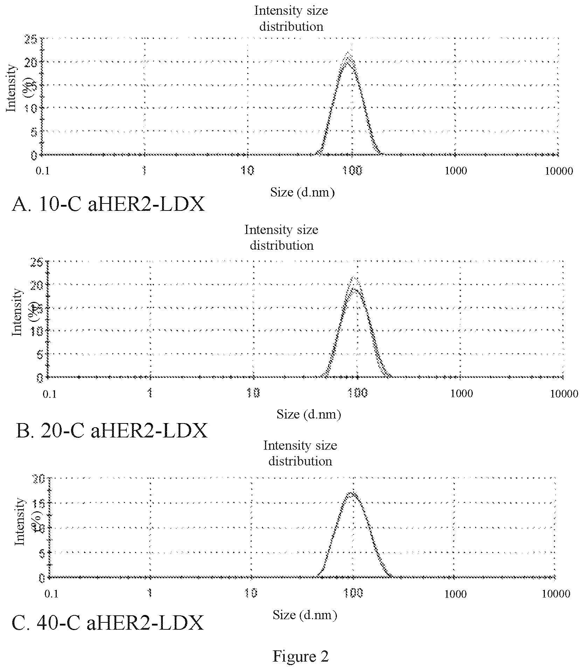

Figures 2A to 2C and Table 2 respectively list the average particle size and PDI of aHER2-LDX with an antibody count of 10, 20 and 40. The average particle size of all three is approximately 80 - 100 nm, and the PDI is less than 0.1. Furthermore, the average particle size is not dependent on the number of antibodies attached to the liposome surface.

Table 2

| Sample Name | Particle Size (nm) | PDI |
|---|---|---|
| 10-C aHER2-LDX | 89.92±1.09 | 0.032 |
| 20-C aHER2-LDX | 92.16±0.33 | 0.046 |

(continued)

| Sample Name | Particle Size (nm) | PDI |
|---|---|---|
| 40-C aHER2-LDX | 95.90±0.55 | 0.093 |
| Note: 10-C aHER2-LDX means that 10 HER2 Fab antibodies are connected to the surface of the doxorubicin-loaded liposome, and so on. | | |

Additionally, the present disclosure provides a method for detecting the post-insertion efficiency of aHER2-DSPE after insertion into the liposome, and the specific procedure is as follows: Sepharose 4B packing was loaded into an 8 mm × 105 mm gel column and the column was equilibrated with PBS buffer. 120 μL of liposomes post-inserted with aHER2-LDX was loaded at the top of the column and the eluent was collected based on the action of gravity. The free aHER2-DSPE and aHER2-LDX liposomes could be completely separated. Quantitative analysis of aHER2-DSPE was performed using RP-HPLC, with the amount of free aHER2-DSPE denoted as $M_{free}$ and the aHER2-DSPE on aHER2-LDX liposomes denoted as $M_{post-insertion}$. The post-insertion efficiency $E_{post-insertion} = M_{post-insertion} / (M_{free} + M_{post-insertion}) * 100\%$.

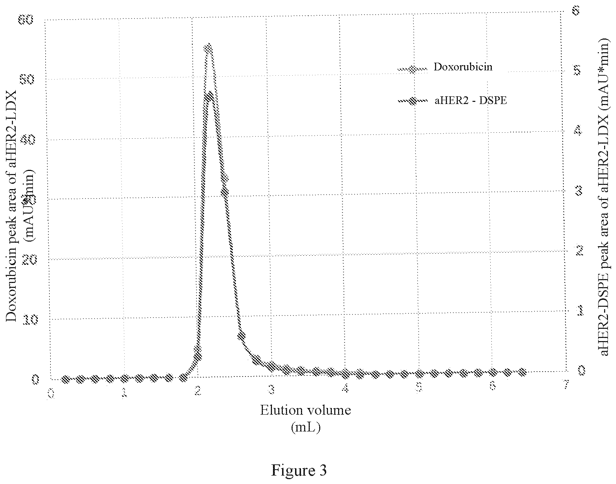

The experimental results showed that the post-insertion efficiency of aHER2-DSPE after insertion into LDX is greater than 95% (as shown in Figure 3), indicating that the post-insertion method provided by the present disclosure for the preparation of a drug-loaded liposome modified with antibodies is excellent.

**3. HER2 Expression in Different Tumor Cell Lines**

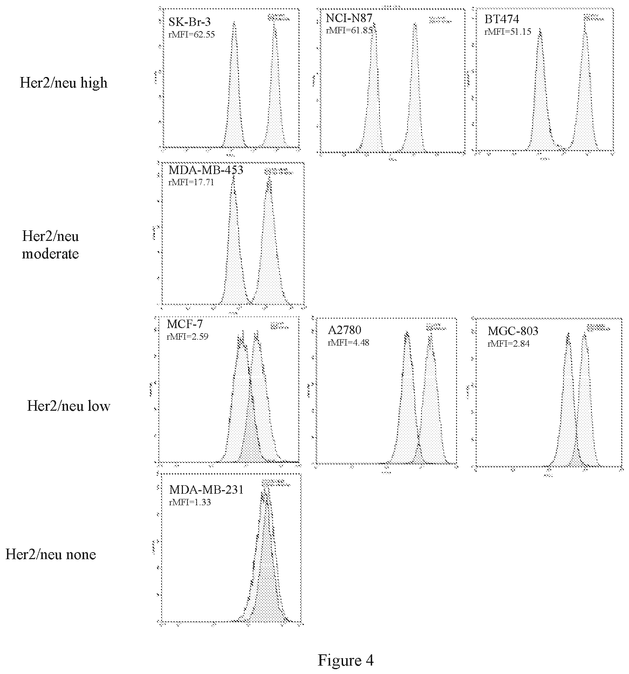

The expression of HER2 antigen on the surfaces of SK-Br-3, BT474, NCI-N87, MDA-MB-453, MCF-7, MDA-MB-231, MGC-803, and A2780 cells was detected using flow cytometry. The results are shown in Figure 4 and Table 3.

Table 3 HER2 Expression Levels on Different Cell Surfaces

| Cell Line | **HER2 Expression (rMFI, FCS)** | Score |
|---|---|---|
| SK-Br-3 | 62.55 | 3+ |
| NCI-N87 | 61.85 | 3+ |
| BT474 | 51.15 | 3+ |
| MDA-MB-453 | 17.71 | 2+ |
| MCF-7 | 2.59 | 1+ |
| A2780 | 4.48 | 1+ |
| MGC-803 | 2.84 | 1+ |
| MDA-MB-231 | 1.33 | 0 |

Remarks: The intensity of HER2 expression is indicated by a + sign, with 0/1+ being low expression. 2+ indicates moderate expression and 3+ indicates high expression. rMFI represents the relative mean fluorescence intensity.

As can be seen from the above experimental data, SK-Br-3, NCI-N87 and BT474 are HER2-overexpressing cell lines; MDA-MB-453 is a HER2-moderately expressing cell line; MCF-7, A2780, MGC-803 and MDA-MB-231 are HER2 low-expressing/negative cell lines.

**4. Formulation Screening for Immunoliposomes Modified with HER2 Antibodies (aHER2-LDX)**

Using the Feedback System Control (FSC) technique as described in the paper "Optimization of drug combinations using Feedback System Control. Nature Protocols. 2016;11:302-315" a total of 10 parameters, including the aHER2 Fab antibody count on the surface of aHER2-LDX (0, 10, 20, 40, 80), LDX formulations (A, B, C, D), and empty liposomes (E), were taken into account in the evaluation of the cytotoxicity ($IC_{50}$) of the drug against HER2-overexpressing and low-expressing cells.

**4.1. Preparation of Different Drug Combinations of aHER2-LDX**

The amounts of aHER2-DSPE and doxorubicin-loaded liposome added in the aHER2-LDX preparation process described above were adjusted to prepare aHER2-LDX with a HER2 Fab antibody count of 0, 10, 20, 40 and 80 on the surface, LDX formulations A, B, C and D, and empty liposomes E. A total of 25 combinations were generated by pairwise combination of these 10 parameters, as follows: A-LDX, B-LDX, C-LDX, D-LDX, empty liposomes E, 10-D aHER2-LDX, 10-C aHER2-LDX, 10-B aHER2-LDX, 10-A aHER2-LDX, 20-D aHER2-LDX, 20-C aHER2-LDX, 20-B aHER2-LDX, 20-A aHER2-LDX, 40-D aHER2-LDX, 40-C aHER2-LDX, 40-B aHER2-LDX, 40-A aHER2-LDX, 80-D aHER2-LDX, 80-C aHER2-LDX, 80-B aHER2-LDX, 80-A aHER2-LDX, 10-E aHER2-LDX, 20-E aHER2-LDX, 40-E aHER2-LDX, 80-E aHER2-LDX (Note: the number in front of the hyphen represents the HER2 Fab antibody count, and the letter after the hyphen represents the different LDX formulations; see Table 1 above for information on the formulations).

**4.2.Cytotoxicity Experiments to Evaluate the Cytotoxic Effect of aHER2-LDX**

The cytotoxic effect of aHER2-LDX on tumor cells was assessed using the CellTiter-Glo® Luminescent Cell Viability Assay kit from Promega Corporation. The specific procedure is as follows: The different tumor cells were digested one day in advance with trypsin, and then seeded into a 96-well clear-bottom black plate at a cell density of 5,000 - 10,000 cells per well in 100 $\mu$L of culture medium. After the cells had fully adhered to the well surface, the drug was added according to the concentration series of 0, 0.25, 0.5, 1, 2, 5, 10, 20, 50, 100 and 150 $\mu$g/mL, with the total volume of the culture medium being, and with three replicate wells prepared for each concentration. The plate was then incubated at 37°C in an incubator for 24 hours. After 24 hours, the drug-containing culture medium was removed, and fresh complete culture medium was added to continue with incubation for 48 hours. The CellTiter-Glo® buffer was prepared in advance and equilibrated to room temperature, while the CellTiter-Glo® lyophilized substrate was also allowed to equilibrate to room temperature. The CellTiter-Glo® buffer was transferred to the CellTiter-Glo® substrate, and the solution was mixed by shaking or inverting to ensure uniformity. The CellTiter-Glo® substrate was completely dissolved within 1 min. The 96-well cell culture plate was then removed from the cell culture incubator and allowed to equilibrate to room temperature for about half an hour, and a control well containing only culture medium (without cells) was prepared to obtain background luminescence. Subsequently, 100 $\mu$L of CellTiter-Glo® reagent was added to each well, and the 96-well cell culture plate was placed on a horizontal shaker to mix the contents for 2 min and promote cell lysis. The plate was then left at room temperature for 10 min until the fluorescent signal stabilized. After the signal had stabilized, luminescence values were recorded using the Spark 10M microplate reader from TECAN Corporation.

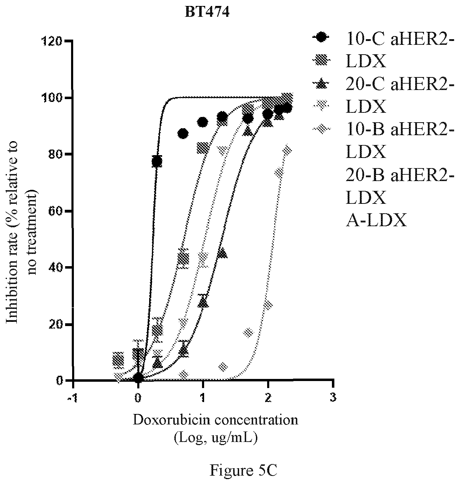

The Graph Prism 8.0 analysis software was used to perform statistical analysis on the recorded luminescence signal values and calculate the $IC_{50}$ values for each drug combination. The results are shown in Tables 4-1, 4-2, and 4-3. Figures 5A, 5B, and 5C respectively illustrate the inhibition of tumor cell growth by 10-C aHER2-LDX, 20-C aHER2-LDX, 10-B aHER2-LDX, 20-B aHER2-LDX, and Formulation A-LDX in the HER2-overexpressing cell lines SK-Br-3, NCI-N87, and BT474.

These data clearly demonstrate the following: HER2 Fab antibodies themselves have minimal cytotoxicity; lowering the drug-to-lipid concentration ratio can enhance cytotoxic effects; the surface modification of LDX with HER2 Fab antibody fragments can further increase drug toxicity to cells. Taken together, the above results show that an antibody count of 10 - 40 is the preferred combination, and furthermore, the drug combination 10-C aHER2-LDX showed the best results. Studies were conducted on cell lines with high, moderate and low HER2 expression, and the results showed that aHER2-LDX exhibited strong cytotoxicity across the entire HER2 expression spectrum, as detailed in Table 4-4.

Table 4-1

| IC$_{50}$ Values for aHER2-LDX Cytotoxicity Against SK-Br-3 (µg/mL) | | | | | |
|---|---|---|---|---|---|
| LDX Formulation / Antibody Count | E | D | C | B | A |
| 0 | ∞ | 3.933 | 6.071 | 11.57 | 84.23 |
| 10 | 182.7 | NA | 0.9742 | 1.332 | 2.352 |
| 20 | 127.4 | 1.823 | 1.423 | 1.201 | NA |
| 40 | 130.5 | 1.501 | 1.468 | 3.071 | NA |
| 80 | 140.4 | NA | NA | 3.387 | 9.527 |

Table 4-2

| IC$_{50}$ Values for aHER2-LDX Cytotoxicity Against BT474 (µg/mL) | | | | | |
|---|---|---|---|---|---|
| LDX Formulation / Antibody Count | E | D | C | B | A |
| 0 | 144585 | 11.32 | 19.46 | 28.04 | 121.4 |
| 10 | 259.6 | NA | 1.718 | 19.49 | 59.8 |
| 20 | 243.5 | 3.146 | 5.088 | 10.74 | NA |
| 40 | 225.5 | 2.867 | 4.594 | 10.44 | NA |
| 80 | 303.3 | NA | NA | 5.879 | 28.2 |

Table 4-3

| IC$_{50}$ Values for aHER2-LDX Cytotoxicity Against NCI-N87 (µg/mL) | | | | | |
|---|---|---|---|---|---|
| LDX Formulation / Antibody Count | E | D | C | B | A |
| 0 | 122813 | 3.673 | 4.687 | 9.435 | 14.86 |
| 10 | 39.59 | NA | 1.143 | 2.669 | 4.341 |
| 20 | 74.14 | 0.80584 | 1.991 | 2.82 | NA |
| 40 | 79.51 | 1.4759 | 1.817 | 3.187 | NA |
| 80 | 83.22 | NA | NA | 3.195 | 2.071 |

Table 4-4

| IC$_{50}$ for Different Tumor Cells (ug/mL) | | | | |
|---|---|---|---|---|
| Drug Combination / Cell Line | Empty Liposome E | 0-A LDX | 0-C LDX | 10-C aHER2-LDX |
| Sk-Br-3 | >1000 | 84.23 | 6.07 | 0.97 |
| BT-474 | >1000 | 121.4 | 19.46 | 1.72 |
| NCI-N87 | >1000 | 14.86 | 4.69 | 1.14 |
| MDA-MB-453 | >1000 | 98.39 | 44.5 | 17.92 |
| MCF-7 | >1000 | 117.2 | 13.06 | 10.10 |
| A2780 | >1000 | 26.69 | 5.51 | 2.14 |
| MGC-803 | >1000 | 31.48 | 3.44 | 1.83 |
| MDA-MB-231 | >1000 | 88.71 | 10.38 | 2.691 |
| MCF-7/Adr | >1000 | 101.3 | 46.41 | 13.70 |

| | | 0 | | |
|---|---|---|---|---|
| | | | | |

**5. Investigation of the Binding Activity of Liposomes with Different Antibody Counts**

The binding activity of immunoliposomes with different antibody counts was analyzed and observed under in vitro conditions with a Gator label-free bioanalyzer using probes with different HER2 antigen levels, and the specific procedure is as follows: (1) the samples to be analyzed and the buffer were added to the corresponding 96-well plate, and the probes were placed in the corresponding well; (2) the temperature of the plate was set to the current temperature of the shaker, and the speed of shaker B was set to 1,000 rpm, so that the new probe is pre-wet in K Buffer for 3 min; (3) test procedure settings: ① Baseline1: K Buffer, 120s; ② Loading: Human Her2 /ErbB2 (23-450) Protein, Fc Tag, 240s; ③ Baseline2: K Buffer, 120s;④ Association: about 200 s for the sample solution to be analyzed (until the binding curve stabilizes); ⑤ Disassociation: about 200 s for K Buffer (until the disassociation curve stabilizes); ⑥ Regeneration: RE Buffer, 5 s, Q Buffer, 5 s, with one cycle for each group, for three cycles in total.

The binding-dissociation curves were fitted and analyzed using the Gator analysis software. The results are shown in Figure 6. From Figure 6, it can be observed that the number of binding liposomes increases with the increase in antigen levels. However, the binding kinetics do not show a strong correlation with lipid concentration and antigen levels. Increasing the antibody count on the liposome surface can accelerate the rate of binding, but it does not have a significant impact on the overall binding capacity.

**6. Investigation of the Binding Ability of aHER2-Lip Conjugated to HER2 Antibodies with Different Numbers of Fab Fragments to Tumor Cells**

Dil fluorescent dye-labeled liposomes were prepared (denoted as Dil-Lip) and were surface-modified with 3, 6, 13, 25, 50, 100, and 200 aHER2 Fab antibody fragments (denoted as aHER2-Dil-Lip-x, where x represents the number of antibody fragments of the modification). Using flow cytometry, the binding to both the HER2-overexpressing cell line SK-Br-3 and the HER2 low-expressing/negative cell line MDA-MB-231 was analyzed.

The prepared aHER2-Dil-Lip liposomes were adjusted to a lipid concentration of 0.5 mg/mL. Subsequently, 10 $\mu$L of the samples were mixed with $2.5 \times 10^5$ SK-Br-3 cells and $2.5 \times 10^5$ MDA-MB-231 cells separately, followed by incubation at 4°C in the dark for 30 min. After incubation, the cells were washed 2 - 3 times with 1 mL of PBS buffer and finally resuspended in 200 $\mu$L of PBS buffer, and analyzed with a flow cytometer.

The results, as shown in Figures 7A and 7B, indicate that when there are only 3 aHER2 Fab antibodies on the liposome surface, aHER2-Dil-Lip exhibits noticeable binding activity with SK-Br-3 cells; when the number of aHER2 Fab antibodies reaches around 13-25, the fluorescence intensity of aHER2-Dil-Lip binding to SK-Br-3 cells tends to be saturated; when the number of aHER2-Dil-Lip surface antibodies is less than 100, there is basically no binding activity with the low-expressing/negative cell line MDA-MB-231, but when the antibody count is greater than 100, there is binding but with weak binding intensity.

### 7. Investigation of Cellular Uptake of aHER2-LDX

The aHER2 Fab antibody fragment was conjugated to doxorubicin liposome and added to the HER2-overexpressing cell line BT474, and the doxorubicin drug content in the cells was detected.

BT474 cells were digested with trypsin one day in advance and spread in a 6-well plate at $5 \times 10^5$ cells/well. C-LDX and 10-C aHER2-LDX were added at the two doses of 10 $\mu$g and 50 $\mu$g, respectively, according to the amount of doxorubicin and blank control wells were set up. After the drug was added, the cells were incubated at 4°C for 4 h; next, the cells were gently washed with PBS buffer 2-3 times, and then scraped off the plate and centrifuged, after which the supernatant was removed. The cells were subjected to three freeze-thaw cycles at -80°C to lyse them. 100 $\mu$L of acetonitrile/methanol (v/v, 1:1) was added for protein precipitation, followed by centrifugation at 10,000 rpm for 10 min. The supernatant was then collected and filtered through a 0.22 $\mu$m organic membrane, and the doxorubicin content was then analyzed by HPLC.

As can be seen in Figure 8, only doxorubicin-loaded liposomes conjugated to HER2 Fab antibody fragments were able to efficiently deliver the drug into the cells, suggesting that aHER2-LDX is able to specifically target HER2-expressing tumor cells and mediate endocytosis.

### 8. Investigation of aHER2-LDX in the Treatment of Tumors with Chemoresistance

The addition of doxorubicin-loaded liposomes conjugated to HER2 antibodies to doxorubicin-resistant cells showed that it can reverse the resistance of tumor cells to chemotherapy drugs.

In order to investigate the performance of HER2 Fab antibody-targeted doxorubicin-loaded liposomes in doxorubicin-resistant tumor cell lines, MCF-7/Adr doxorubicin-resistant breast cancer cells were selected for testing. The cytotoxic effect of HER2/neu antibody-targeted doxorubicin-loaded liposomes on tumor cells was assessed using the CellTiter-Glo® Luminescent Cell Viability Assay kit from Promega Corporation, with trastuzumab used as a control, and with concurrent comparisons made against the non-targeted Formulations C and A doxorubicin-loaded liposomes. The Graph Prism 8.0 analysis software was used to fit the cytotoxicity curves and calculate the individual $IC_{50}$ values. The results are shown in Figure 9.

The cytotoxicity results show that the $IC_{50}$ values of HER2 Fab antibody-targeted doxorubicin-loaded liposomes, Formulation C and Formulation A doxorubicin-loaded liposomes, as well as trastuzumab in MCF-7/Adr are 13.70 $\mu$g/mL, 46.41 $\mu$g/mL, and 101.30 $\mu$g/mL, respectively. This indicates that HER2 Fab antibody-targeted doxorubicin-loaded liposomes still exhibit strong cytotoxicity against doxorubicin-resistant cell lines. Therefore, they have potential efficacy in the treatment of patients with doxorubicin-resistant tumors in clinical practice.

### 9. Investigation of In Vivo Pharmacodynamics of aHER2-LDX Conjugated to Different Amounts of HER2 Antibodies in Animals

#### 9.1. Construction of BT474 Breast Cancer Balb/C-Nude Nude Mouse Xenograft Model

Construction of BT474 breast cancer Balb/C-Nude nude mouse xenograft model: 4 - 5 weeks old female Balb/C-Nude nude mice, weighing about 18 g and of SPF grade were purchased and allowed to acclimatize in the animal breeding room for about a week. After BT474 cells grew to the logarithmic growth phase, they were digested with trypsin, centrifuged, and then collected. The cells were then washed with PBS buffer 2-3 times, then resuspended in PBS buffer and counted. The cell density was then adjusted to 1 * 10^8 cells/mL. The Matrigel matrix gel was removed from the refrigerator at -20°C and melted on ice in advance, and the cell suspension was mixed with the Matrigel matrix gel according to a volume of 1:1. Based on the amount of 5 * 10^6 - 1 * 10^7 cells, a 1 mL syringe was used to aspirate the corresponding volume of cell suspension and subcutaneously inoculate it into the right axillary area of nude mice, with pressure applied to the needle puncture site for 30 s after inoculation to prevent the leakage of the cell solution. After inoculation, a significant subcutaneous papule was observed. The inoculated mice were observed regularly and tumor growth was monitored.

## 9.2. Dosing and Efficacy Evaluation

When the tumor size reached 100 - 200 mm$^3$, the mice were randomly divided into six groups, with at least six mice in each group, and the six groups were: the control group, the Formulation A doxorubicin-loaded liposome group, the Formulation C doxorubicin-loaded liposome group, and the HER2 Fab antibody-targeted Formulation C doxorubicin-loaded liposome groups with antibody counts of 10, 20, and 40 (i.e., the 10-C aHER2-LDX group, the 20-C aHER2-LDX group, and the 40-C aHER2-LDX group).

After grouping, the control group was given empty liposomes via the caudal vein. The nude mice in the Formulation A doxorubicin-loaded liposome group were given a 2 mg/kg dose of Formulation A doxorubicin-loaded liposome via the caudal vein according to body weight; the nude mice in the Formulation C doxorubicin-loaded liposome group were also given a 2 mg/kg dose of Formulation C doxorubicin-loaded liposome via the caudal vein according to body weight; the nude mice in the 10-C aHER2-LDX group, 20-C aHER2-LDX group, and 40-C aHER2-LDX group were given a 2 mg/kg dose of HER2 Fab antibody-targeted Formulation C doxorubicin liposome via the caudal vein according to body weight. The drug was administered once a week and a total of 5 doses were administered. The body weight and tumor growth curve of the mice were recorded every other day. The mice were euthanized after the end of dosing (the control group was euthanized on Day 18 due to excessive tumor volume).

The inhibitory effect and complete curative ability of the test article on breast cancer BT474 mouse xenografts were mainly tested.

Measurement of tumor volume and weight of tumor-bearing mice: Measurements were taken every other day using vernier calipers Tumor volume was calculated as $V = 0.5 \, a \times b^2$, where a and b represent the length and width diameters of the tumor, respectively.

Tumor growth inhibition rate TGI (%) = [1 - (Di - D0) / (Ci - C0) $\times$100, of which Di - $D_0$ > 0. $D_0$ is the mean tumor volume at the first dose in the dosing group (Drug), $D_i$ is the mean tumor volume at a certain measurement after the start of dosing in the dosing group; $C_0$ is the mean tumor volume at the first dose in the control group (Control), and $C_i$ is the mean tumor volume at a certain measurement after the start of dosing in the control group.

The in vivo pharmacodynamic results in BT474 breast cancer Balb/C-Nude nude mouse xenografts are shown in Figure 10. The results show that the tumor growth inhibition rate (TGI%) of Formulation A doxorubicin-loaded liposome, Formulation C doxorubicin-loaded liposome, and 10-C aHER2-LDX, 20-C aHER2-LDX, and 40-C aHER2-LDX on Day 18 after dosing was 50.55%, 60.12%, 69.66%, 61.98%, and 62.94%, respectively, indicating that HER2 Fab antibody-targeted Formulation C doxorubicin-loaded liposome had a stronger ability to inhibit tumor growth than Formulation C doxorubicin-loaded liposome and Formulation A doxorubicin-loaded liposome. However, increasing the number of doxorubicin-loaded liposome surface antibodies did not further enhance the tumor inhibitory effect, and these results were consistent with those of the cytotoxicity experiment. These experimental results clearly demonstrate the following: 1) doxorubicin-loaded liposomes with reduced lipid ratio not only exhibit stronger cytotoxic effects on tumor cells in vitro but also show superior tumor growth inhibition in vivo; 2) additionally, HER2 Fab antibody-targeted Formulation C doxorubicin-loaded liposomes further enhance the capability to inhibit tumor growth, indicating that surface modification with antibodies on doxorubicin-loaded liposomes can facilitate targeting and improve the effectiveness of tumor inhibition.

## 10. Investigation of In Vivo Dose-Dependent Efficacy of aHER2-LDX in Animals

## 10.1. Construction of NCI-N87 Breast Cancer Balb/C-Nude Nude Mouse Xenograft Model

The NCI-N87 gastric cancer cell line with high cell-surface HER2 expression was selected to construct a Balb/C-Nude nude mouse xenograft model.

4 - 5 weeks old female Balb/C-Nude nude mice, weighing about 18 g and of SPF grade were purchased and allowed to acclimatize in the animal breeding room for about a week. After NCI-N87 cells grew to the logarithmic growth phase, they were digested with trypsin, centrifuged, and then collected. The cells were then washed with PBS buffer 2-3 times, then resuspended in PBS buffer and counted. The cell density was then adjusted to 2 * 10^8 cells/mL. The Matrigel matrix gel was removed from the refrigerator at -20°C and melted on ice in advance, and the cell suspension was mixed with the Matrigel matrix gel according to a volume of 1:1. Based on the amount of 1 * 10^7 cells, a 1 mL syringe was used to aspirate the corresponding volume of cell suspension and subcutaneously inoculate it into the right axillary area of nude mice, with pressure applied to the needle puncture site for 30 s after inoculation to prevent the leakage of the cell solution. After inoculation, a significant subcutaneous papule was observed. The inoculated mice were observed regularly and tumor growth was monitored.

**10.2. Dosing and Efficacy Evaluation**

When the tumor size reached 100 - 200 mm$^3$, the mice were randomly divided into seven groups, with at least six mice in each group, and the seven groups were: the control group, the trastuzumab group, the Formulation A doxorubicin-loaded liposome group, and the HER2 Fab antibody-targeted Formulation C doxorubicin-loaded liposome (10-C aHER2-LDX) groups with doses of 0.5, 2, 5, and 10 mg/kg.

After grouping, the control group was given empty liposomes via the caudal vein. The nude mice in the Formulation A doxorubicin-loaded liposome group were given a 2 mg/kg dose of doxorubicin-loaded liposome with a drug-to-lipid ratio of 0.17 via the caudal vein according to body weight; the nude mice in the 10-C aHER2-LDX group were also similarly given 0.5, 2, 5 and 10 mg/kg doses of HER2 Fab antibody-targeted Formulation C doxorubicin-loaded liposome via the caudal vein according to body weight; at the same time, the trastuzumab group was set as the control group and given 5 mg/kg once a week and a total of 5 doses were administered. The body weight and tumor growth curve of the mice were recorded every other day, and the mice were euthanized after the end of dosing.

The inhibitory effect and complete curative ability of the test article on the NCI-N87 gastric cancer xenograft model in mice were mainly tested.

(1) Measurement of tumor volume: Measurements were taken every other day using vernier calipers Tumor volume was calculated as V = 0.5 a $\times$ b$^2$, where a and b represent the length and width diameters of the tumor, respectively.

(2) Tumor growth inhibition rate TGI (%) = [1 - (D$_i$ - D$_0$) / (C$_i$ - C$_0$] $\times$ 100, of which D$_i$ - D$_0$ > 0. D$_0$ is the mean tumor volume at the first dose in the dosing group (Drug), D$_i$ is the mean tumor volume at a certain measurement after the start of dosing in the dosing group; C$_0$ is the mean tumor volume at the first dose in the control group (Control), and C$_i$ is the mean tumor volume at a certain measurement after the start of dosing in the control group.

The in vivo pharmacodynamic results for NCI-N87 gastric cancer Balb/C-Nude nude mice xenografts are shown in Figure 11. The tumor inhibition rates of Formulation A doxorubicin-loaded liposome, trastuzumab and 10-C aHER2-LDX at doses of 0.5, 2, 5, and 10 mg/kg, respectively, were 77.27%, 14.49%, 28.41%, 81.71%, 88.75%, and 96.33%, respectively. This result indicates that the doxorubicin-loaded liposome conjugated to 10 HER2 Fab antibodies has a better tumor growth inhibitory effect in vivo on the HER2-overexpressing NCI-N87 gastric cancer tumor model and exhibits a dose-dependent relationship.

**11. Investigation of In Vivo Pharmacodynamics of aHER2-LDX in An Animal Model of HER2-Low Expressing Tumors**

**11.1. Construction of MCF-7 Breast Cancer Balb/C-Nude Nude Mouse Xenograft Model**

The MCF-7 breast cancer cell line with low cell-surface HER2 expression was selected to construct a Balb/C-Nude nude mouse xenograft model.

The tumor model construction method was the same as that in 10.1. Construction of NCI-N87 Breast Cancer Balb/C-Nude Nude Mouse Xenograft Model

**11.2. Dosing and Efficacy Evaluation**

When the tumor size reached 100 - 200 mm$^3$, the mice were randomly divided into six groups, with at least six mice in each group, and the six groups were: the control group, the trastuzumab group, the Formulation A doxorubicin-loaded liposome group, and the HER2 Fab antibody-targeted Formulation C doxorubicin-loaded liposome (10-C aHER2-LDX) groups with doses of 2, 5, and 10 mg/kg.

After grouping, the control group was given empty liposomes via the caudal vein. The nude mice in the Formulation A doxorubicin-loaded liposome group were given a 2 mg/kg dose of doxorubicin-loaded liposome with a drug-to-lipid ratio of 0.17 via the caudal vein according to body weight; the nude mice in the 10-C aHER2-LDX group were also similarly given 2, 5 and 10 mg/kg doses of HER2 Fab antibody-targeted Formulation C doxorubicin-loaded liposome via the caudal vein according to body weight; at the same time, the trastuzumab group was set as the control group and given 5 mg/kg once a week and a total of 5 doses were administered. The body weight and tumor growth curve of the mice were recorded every other day, and the mice were euthanized after the end of dosing.

The inhibitory effect and complete curative ability of the test article on breast cancer MCF-7 mouse xenografts were mainly tested.

The efficacy evaluation method was the same as that in 10.2.

The in vivo pharmacodynamic results for MCF-7 breast cancer Balb/C-Nude nude mouse xenografts are shown in Figure 12. The tumor inhibition rates of trastuzumab, Formulated A doxorubicin-loaded liposome and 10-C aHER2-LDX at doses of 2, 5 and 10 mg/kg, respectively, are 2.91%, 62.16%, 69.67%, 92.18%, and 97.38%. This result similarly indicates that the doxorubicin-loaded liposome conjugated to 10 HER2 Fab antibodies has a better tumor growth inhibitory effect in vivo on the HER2-low expressing MCF-7 breast cancer tumor model and exhibits a dose-dependent relationship. Inference from the results: the doxorubicin-loaded liposome modified with HER2 Fab antibodies has the same treatment effect in patients with HER2-low expressing tumors as in patients with HER2-overexpressing tumors, with a potential broader range of indications.

## 12. Investigation of In Vivo Pharmacokinetic Characteristics of aHER2-LDX in Animals

The in vivo pharmacokinetic characteristics of the doxorubicin-loaded liposome with different antibody counts were investigated in tumor-bearing mouse models.

The method for constructing the BT474 breast cancer Balb/C-Nude nude mouse xenograft model is as described in Embodiment 9.1.and grouping and dosing were carried out when the subcutaneous tumor volume reached 400 - 600 mm$^3$

Dosing: The mice in the first group were given 10-C aHER2-LDX via the caudal vein according to body weight, and the drug dose was 5 mg/kg; the mice in the second group were given 10-C aHER2-LDX via the caudal vein according to body weight, and the drug dose was 5 mg/kg.Blood collection: Blood was collected at 0.5, 1, 2, 4, 6, 8, 12, 24, 48 and 72 hours after dosing in EDTA anticoagulant tubes, which were placed on ice, and centrifuged at a rotational speed of 3,000 rpm at 4°C for 30 min. The upper layer of plasma was then carefully pipetted into a new, labeled centrifuge tube, and subsequently tested or stored in a -80°C refrigerator.

Detection of doxorubicin drug content in plasma: 40 $\mu$L of plasma sample was accurately measured and placed in a centrifuge tube, added with 160 $\mu$L of acetonitrile-methanol (1:1, v/v) according to a ratio of 1:4 for plasma to organic reagent, vortexed for 1 - 2 to fully precipitate the protein in the plasma, then centrifuged at a rotational speed of 1,000 $\times$ g at 4°C for 10 min. The supernatant was then carefully pipetted into a new centrifuge tube, taking care to avoid aspirating the protein layer, centrifuged at a rotational speed of 1,000 $\times$ g at 4°C for 10 min, and filtered with a 0.22 $\mu$m organic filter head, after which 50 uL was placed in a sample vial, and HPLC detection was performed. High performance liquid chromatography conditions (with reference to the Chinese Pharmacopoeia): The Athena C18 column (120A, 4.6150 mm, 5 $\mu$m) with octadecyl silane bonded to silica gel as filler was used; sodium dodecyl sulfate solution (1.44 g of sodium dodecyl sulfate and 0.68 mL of phosphoric acid were added with 500 mL of water for dissolution)-acetonitrile-methanol (500:500:60) was used as the mobile phase; the detection wavelength was 254 nm; the detection flow rate was 1.0 ml/min; the sample volume was 10 $\mu$L. $\times$

Detection of HER2 Fab antibody content in plasma:The content of HER2 Fab antibodies in plasma was detected by the ELISA method. HER2 antigen was coated in a 96-well microplate one day in advance at 4°C. 350 $\mu$L of cleaning solution was added to each well for washing three times, and 300 $\mu$L of blocking solution was added to each well [for blocking at] 37°C for 1 h. 350 $\mu$L of cleaning solution was then added to each well for washing three times. The reference standard, sample or control article was then added to the corresponding well, at 100 $\mu$L/well, and the reaction wells were sealed with plate sealer. Incubation was then performed at 37°C with shaking for 1 h, after which 350 $\mu$L of cleaning solution was added to each well for washing three times. Following this, chromogenic substrate was added at 100 $\mu$L/well and incubation was performed in the dark at room temperature for 3 - 15 min. Finally, the stop solution was added at 100 $\mu$L/well and mixed well, and the OD450 value was immediately measured.

Analysis: The plasma concentration results of the doxorubicin-loaded liposome with different amounts of HER2 Fab antibodies in BT474 tumor-bearing mice are shown in Figure 13A, and the changes in HER2 Fab antibody concentration in blood samples are shown in Figure 13B. Changes in drug concentration and HER2 Fab antibodies in the blood can be used to indicate the process of drug release from the HER2 Fab antibody-targeted liposomes. It can be observed that liposomes with different antibody densities have different in vivo release processes. Liposomes with low antibody density exhibit slower drug release, while HER2 Fab release is faster.

## 13. Formulation Screening for Immunoliposomes Modified with CD3 $\times$ HER2 Dual-Targeting Antibodies (CD3 $\times$ HER2 Lip)

### 13.1. Preparation of Immunoliposomes Modified with CD3 $\times$ HER2 Dual-Targeting Antibodies (CD3 $\times$ HER2 Lip)

An empty liposome was prepared with reference to Embodiment 1. 900 mg of HSPC, 300 mg of Chol, and 60 mg of DSPE-mPEG2000 were added to 2 mL of anhydrous ethanol and heated in a water bath to 60°C. Then, a magnetic stirrer was added for stirring until complete dissolution, resulting in a clear liquid mixture, which was the lipid mixture. At the same time, 3.304 g of ammonium sulfate was placed in a glass bottle, added with 100 mL of ultrapure water, and stirred until dissolution, and the pH was then adjusted to 5.0 to obtain an ammonium sulfate aqueous solution with a concentration of 250 mM; this solution was prepared just before use. The dissolved lipid mixture was injected into 19 mL of the ammonium sulfate aqueous solution using a syringe, then stirred and extruded six times, and dialysis was then performed using a 10 kD dialysis membrane in a HEPES buffer (10 mM, pH 6.8) to obtain an empty liposome with an external aqueous phase of a HEPES solution.

The C of the CD3 Fab antibody at position 231 of the heavy chain contains a free thiol group, and this free thiol group (-SH) is exposed by reduction at a specific site. The free thiol group chemically reacts with the maleimide group of the lipid molecule DSPE-PEG-MAL to give the CD3 Fab antibody fragment-lipid molecule (denoted as follows: aCD3-DSPE). Refer to the preparation of aHER2-DSPE in Embodiment 2.1 for the specific procedure.

In order to prepare liposomes with different combinations of CD3 antibodies and HER2 antibodies at varying densities on the surface, the amounts of aHER2-DSPE and aCD3-DSPE added were adjusted and they were co-incubated with empty liposome to prepare liposomes modified with different densities of CD3 × HER2 dual-targeting antibodies (denoted as CD3 × HER2 Lip a × b, where 'a' represents the CD3 Fab antibody count, and 'b' represents the HER2 Fab antibody count, namely CD3 × HER2 Lip 9x5, CD3 × HER2 Lip 9x10, CD3 × HER2 Lip 9x20, CD3 × HER2 Lip 9x40, CD3 × HER2 Lip 9x80, as well as CD3 × HER2 Lip 3x10, CD3 × HER2 Lip 6x10, CD3 × HER2 Lip 18x10, and CD3 × HER2 Lip 36x10. Single-targeting liposomes with HER2 antibodies and CD3 antibodies were also prepared separately to give HER2 Lip-5, HER2 Lip-10, HER2 Lip-20, HER2 Lip-40, HER2 Lip-80, as well as CD3 Lip-3, CD3 Lip-6, CD3 Lip-9, CD3 Lip-18, and CD3 Lip-36.

### 13.2. In Vitro Evaluation of Immunoliposomes Modified with CD3 × HER2 Dual-Targeting Antibodies (CD3 × HER2 Lip)

The ability of CD3 × HER2 dual-targeting liposomes to activate Jurkat cells in vitro was investigated using CD3-positive Juurkat [sic: Jurkat] cells as a representative.

Jurkat, Clone E6-1 human T-lymphoblastic leukemia cell line was obtained from the College of Pharmaceutical Sciences of Shanghai Jiao Tong University. The cell line was grown in RPMI-1640 complete culture medium containing 1% penicillin-streptomycin dual antibiotics and 10% fetal bovine serum.

The expression of CD25 and CD69 on the surface of CD3-positive Jurkat cells was analyzed by flow cytometry to investigate the in vitro activation of T cells by CD3 × HER2 dual-targeting antibody liposomes and to select the optimal antibody density. Jurkat cells were resuspended in complete culture medium at $1 \times 10^5$ Jurkat cells per well, with a culture medium volume of 200 µL, and added to a 96-well plate. 0.01 µg, 0.1 µg, 1 µg, 5 µg, and 10 µg of CD3 × HER2 dual-targeting liposomes, CD3 single-targeting liposomes, or HER2 single-targeting liposomes were added to each well. Incubation was continued for 48 h in a 37°C incubator. Subsequently, the 96-well cell culture plate was removed, centrifuged for 5 min at 400 g, and the supernatant was discarded. 100 µL of PBS buffer was then added to resuspend the cells, and anti-human CD25 PE-Cy7 and anti-huamn [sic: anti-human] CD69 PE fluorescent antibodies were added. The cells were incubated at room temperature for 15 min and then washed twice with PBS buffer, × Finally, the cells were resuspended in 100 µL of PBS buffer and loaded and tested with the Novocyte flow cytometer. The results are shown in Figures 14A and 14B.

The ability of CD3 × HER2 dual-targeting liposomes to activate T cells in vitro and to mediate effector cell killing of target cells in vitro was investigated using CD3/CD28 antibody-stimulated expanded T cells as a representative.

The cytotoxic effect of CD3 × HER2 dual-targeting antibody liposomes in mediating effector cell killing of target cells in vitro was assessed using the CytoTox96® Non-Radioactive Cytotoxicity Assay kit from Promega. At the same time, the expression of CD25 and CD69 on the surface of T cells was analyzed by flow cytometry to investigate the in vitro activation of T cells by CD3 × HER2 dual-targeting liposomes and to select the optimal antibody density. The effector cells (T cells stimulated and expanded with CD3/CD28 antibodies) and the target cells SK-Br-3 were resuspended in phenol red-free RPMI-1640 complete culture medium, at cell concentrations of $1.11 \times 10^6$ cells/mL and $1.0 \times 10^5$ cells/mL, respectively. The cells were plated at a ratio of 10:1 for effector cells to target cells, with 90 µL of effector cells and 100 µL of target cells added to a U-bottom 96-well cell culture plate and mixed thoroughly. 10 µL of different doses of the drug, namely 0.01 µg, 0.1 µg, 1 µg, 5 µg, and 10 µg of CD3 × HER2 dual-targeting liposomes, CD3 single-targeting liposomes, or HER2 single-targeting liposomes was added to each well. The 96-well plate was placed in a 37°C carbon dioxide incubator and incubated for 48 h. After the incubation, the 96-well culture plate was removed and centrifuged at 250 × g for 5 min. Following centrifugation, 50 µL of the supernatant was aspirated and transferred to a new 96-well cell culture plate. Subsequently, 50 µL of the substrate reaction mixture was added to the new plate, and the plate was covered with aluminum foil or an opaque box to shield it from light and incubated at room temperature for 30 min. Then, 50 µL of stop solution was added, and the absorbance value was measured at 490 nm using a microplate reader. The results are shown in Figure 15.

The lower layer of cells after the supernatant removal as described above was washed twice with PBS buffer, and added with anti-human CD4 APC-Cy7, anti-human CD8 FITC, anti-human CD25 PE-Cy7 and anti-huamn [sic: anti-human] CD69 PE fluorescent antibodies, then incubated for 15 min at room temperature, after which the cells were washed twice with PBS buffer. Finally, the cells were resuspended in 100 µL of PBS buffer and loaded and tested with the Novocyte flow cytometer. The results are shown in Figures 16A and 16B.

Summarizing the results of Figure 14, Figure 15 and Figure 16 above, it can be concluded that CD3 × HER2 dual-targeting liposomes have a significant activation effect on both CD25 and CD69 within 48 h, and a significant cell killing effect within 48 h. When the CD3 antibody count on the liposome surface was 9, the expression of CD25/CD69 molecules decreased with the increase in the HER2 antibody count; when the HER2 antibody count on the liposome surface was 10, the expression of CD25/CD69 molecules gradually increased with the increase in the CD3 antibody count, indicating that the activation effect was more significant.

Overall, it can be seen that in CD3 × HER2 dual-targeting liposomes, a higher HER2 antibody count does not necessarily lead to better results. The optimal activation of T cells is achieved with an antibody count of 5 -10, and when considering the cytotoxic effect, the ideal HER2 antibody count is 10. The higher the density of CD3 antibodies, the better the T cell activation effect, but on the one hand, a plateau is reached when the CD3 antibody count reaches 9 or 18, and on the other hand, higher T cell immune activation is not necessarily better in vivo.

### 14. Preparation of Immunoliposomes Modified with CD3 × HER2 Dual-Targeting Antibodies (CD3 × HER2 R848-Lip)

285.7 mg of HSPC, 95 mg of Chol, and 19 mg of DSPE-mPEG2000 were added to 1 mL of anhydrous ethanol and heated in a water bath to 60°C. Then, a magnetic stirrer was added for stirring until complete dissolution, resulting in a clear liquid mixture. At the same time, 6.607 mg of ammonium sulfate was placed in a glass bottle, added with 100 mL of ultrapure water, and stirred until dissolution, and the pH was then adjusted to 5.0 to obtain an ammonium sulfate aqueous solution with a concentration of 500 mM; this solution was prepared just before use. The dissolved lipid mixture was injected into 9 mL of the ammonium sulfate aqueous solution using a syringe, stirred while hydrating at 60°C for 45 min, then extruded six times with an extruder, and dialysis was then performed using a 10 kD dialysis membrane in a dialysate (10 mM HEPES, 0.9% NaCl, pH 5.5) to obtain an empty liposome.

Another 6.25 mL of 1 mg/mL R848 solution was added to 3.75 mL of empty liposomes with a lipid concentration of 40 mg/mL, making a total volume of 10 mL. The mixture was stirred at room temperature for 30 min for drug loading, after which a 100 kD dialysis bag was used to remove free drug, giving the prepared R848 liposomes. The particle size is 80 - 120 nm, with a PDI of less than 0.1.

With reference to the preparation of aHER2-DSPE in Embodiment 2.1 and the preparation of aCD3-DSPE in Embodiment 13.1, aHER2-DSPE and aCD3-DSPE were added to the above R848 liposomes in a 60°C water bath and incubated for 10 - 30 min, with a ratio of 10 HER2 antibodies and 6 CD3 antibodies on the surface of each liposome, and then ultrafiltration and dialysis were performed.

### 15. Evaluation of In Vivo Pharmacodynamics of Immunoliposomes (CD3 × HER2 Lip) and R848 Liposomes (CD3 × HER2R848-Lip) Modified with CD3 × HER2 Dual-Targeting Antibodies

#### 15.1. Construction of Humanized NCG-B2M-KO Mouse Xenograft NCI-N87 Breast Cancer Model

The NCI-N87 gastric cancer cell line with high cell-surface HER2 expression was selected to construct a humanized model of NCG-B2M-KO severely immunodeficient mice.

4 - 5 weeks old female Balb/C-Nude nude mice, weighing about 18 g and of SPF grade were purchased and allowed to acclimatize in the animal breeding room for about a week. After NCI-N87 cells grew to the logarithmic growth phase, they were digested with trypsin, centrifuged, and then collected. The cells were then washed with PBS buffer 2-3 times, then resuspended in PBS buffer and counted. The cell density was then adjusted to 2 * 10^8 cells/mL. The Matrigel matrix gel was removed from the refrigerator at -20°C and melted on ice in advance, and the cell suspension was mixed with the Matrigel matrix gel according to a volume of 1:1. Based on the amount of 1 * 10^7 cells, a 1 mL syringe was used to aspirate the corresponding volume of cell suspension and subcutaneously inoculate it into the right axillary area of nude mice, with pressure applied to the needle puncture site for 30 s after inoculation to prevent the leakage of the cell solution. After inoculation, a significant subcutaneous papule was observed. The inoculated mice were observed regularly and tumor growth was monitored. At the same time, each mouse was intravenously injected with 1 * 10^7 PBMC cells from healthy volunteers via the caudal vein.

#### 15.2. Dosing and Efficacy Evaluation

When the tumor size reached 100 - 200 mm$^3$ and after successful humanization of the mice, the mice were randomly divided into seven groups, with at least six mice in each group, and the seven groups were: the empty group (inoculated with tumor cells only), the control group (inoculated with tumor cells + PBMC cells), R848 liposome group, CD3 × HER2 double-targeting liposome group at **doses** of 0.1, 1 and 2 mg/kg, and CD3 × HER2 double-targeting R848 liposome group at 0.1 mg/kg.

After grouping, the empty group and the control group were intravenously injected with the PBS solution via the caudal vein. The nude mice in the CD3 × HER2 dual-targeting liposome group were given 0.1, 1 and 2 mg/kg of CD3 × HER2 dual-targeting liposomes via the caudal vein according to body weight; the nude mice in the CD3 × HER2 dual-targeting R848 liposome group were also similarly given 0.1 mg/kg of CD3 × HER2 dual-targeting R848 liposome via the caudal vein according to body weight. Dosing was performed twice a week, for a total of 7 doses. The body weight and tumor growth curve of the mice were recorded every other day, and the mice were euthanized after the end of dosing.

The inhibitory effect and complete curative ability of the test article on the NCI-N87 gastric cancer xenograft model in mice were mainly tested. Refer to Embodiment 10.2 for the tumor volume measurement and tumor growth inhibition rate calculation.

The in vivo pharmacodynamic results for the humanized NCG-B2M-KO mouse xenograft NCI-N87 breast cancer model are shown in Figure 17. CD3 × HER2 dual-targeting liposomes have a significant inhibitory effect on tumor growth, and the anti-tumor effect can be further improved when the immunomodulatory drug R848 is encapsulated within the liposomes.

All literature mentioned in the present disclosure is cited as a reference in the present application, as if each piece of literature is cited separately as a reference. It should also be understood that, after reading the above description of the contents of the present disclosure, a person skilled in the art may make various changes or modifications to the present disclosure, and such equivalents similarly fall within the same scope defined by the claims appended to the present application.

**Claims**

1.  A nanoparticle, **characterized in that** the surface of the nanoparticle contains antibodies; the number of the antibodies is 5 - 60, preferably 5 - 50, and more preferably 5-40.

2.  The nanoparticle according to Claim 1, **characterized in that** the number of the antibodies is 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39 or 40.

3.  The nanoparticle according to Claim 1, **characterized in that** the antibodies comprise HER2 antibodies (human epidermal growth factor receptor 2 antibodies).

4.  The nanoparticle according to Claim 1, **characterized in that** the nanoparticle is a liposome or nanosized particle.

5.  The nanoparticle according to Claim 1, **characterized in that** the antibodies further comprise a second antibody, and the two antibodies are of different types.

6.  The nanoparticle according to Claim 5, **characterized in that** the number of the second antibody is 0 - 60, preferably 3 - 36.

7.  The nanoparticle according to Claim 5, **characterized in that** the second antibody comprises CD3 antibodies (human cluster of differentiation 3 antibodies).

8.  The nanoparticle according to Claim 1, **characterized in that** the number of antibodies on the surface of the nanoparticle is calculated based on the following formula:

$$P = \frac{\frac{Y}{L} * NA}{N} * X * \frac{1}{NA} * MW = \frac{X * Y * MW}{N * L} \; ;$$

where X is the number of antibodies contained on the surface of each liposome;
MW is the average relative molecular mass of the protein;
Y is the lipid mass;
L is the average relative molecular mass of the lipids;
N is the average number of lipid molecules contained in each liposome; NA is the Avogadro constant;

P is the amount of antibodies required to prepare each liposome with X antibody molecules on the surface.

9. The nanoparticle according to Claim 4, **characterized in that** the liposome is a drug-loaded liposome in which the weight ratio (drug-to-lipid ratio) of the drug to the lipid material is 1:10-100.

10. The nanoparticle according to Claim 4, **characterized in that** the liposome is a drug-loaded liposome in which the weight ratio (drug-to-lipid ratio) of the drug to the lipid material is 1:25-35, preferably 1:28-32.

11. The nanoparticle according to Claim 9, **characterized in that** the drug comprises an anti-tumor drug which is selected from the following group: anthracyclines, platinum-based drugs, fluorouracils, camptothecins, taxanes, immunomodulatory drugs, or combinations thereof, preferably doxorubicin or resiquimod (R848).

12. The nanoparticle according to Claim 1, **characterized in that** the modification of the nanoparticle with antibodies is by the post-insertion method.

13. A composition, **characterized in that** the composition comprises the nanoparticle according to Claim 1.

14. The use of the nanoparticle according to Claim 1 or the composition according to Claim 13, **characterized in that** it is for the preparation of drugs used in the prevention and/or treatment of tumors.

15. The nanoparticle according to Claim 14, **characterized in that** the tumors are selected from the following group: breast cancer, gastric cancer, ovarian cancer, liver cancer, or combinations thereof.

16. The nanoparticle according to Claim 14, **characterized in that** the tumors comprise tumors that have multidrug resistance to antitumor drugs.

17. A method for the prevention and/or treatment of tumors, **characterized in that** the nanoparticle according to Claim 1 or the composition according to Claim 13 is administered to a subject in need.

**HER2 Fab:DSPE-PEG2000-MAL ratio**

Figure
1 A

Figure
1B

Figure 1 C

aHER2-DSPE

Min

Figure 1 D

Intensity size
distribution

A. 10-C aHER2-LDX

Intensity size
distribution

B. 20-C aHER2-LDX

Intensity size
distribution

C. 40-C aHER2-LDX

Figure 2

Figure 3

Her2/neu high

SK-Br-3
rMFI=62.55

NCI-N87
rMFI=61.85

BT474
rMFI=51.15

Her2/neu
moderate

MDA-MB-453
rMFI=17.71

Her2/neu low

MCF-7
rMFI=2.59

A2780
rMFI=4.48

MGC-803
rMFI=2.84

Her2/neu none

MDA-MB-231
rMFI=1.33

Figure 4

**SK-BR-3**

Figure 5A

**NCI-N87**

Figure 5B

Figure 5C

Figure 6

**Sk-Br-3**

Figure 7A

**MDA-MB-231**

Figure 7B

Figure 8

**MCF-7/Adr**

Figure 9

**BT474 xenograft model**

Figure 10

**NCI-N87 xenograft model**

Figure 11

## MCF-7 xenograft

Figure 12

Figure 13A

**B**

Figure 13B

Figure 14A

**Jurkat-48h**

Figure 14B

**48h**
**T cells: SK-Br-3**
**E:T=10:1**

Figure 15

Figure 16

Figure 17

## INTERNATIONAL SEARCH REPORT

| International application No. |
|---|
| **PCT/CN2022/094222** |

**A.    CLASSIFICATION OF SUBJECT MATTER**

A61K 47/68(2017.01)i;  A61K 47/69(2017.01)i;  A61K 31/704(2006.01)i;  A61P 35/00(2006.01)i

According to International Patent Classification (IPC) or to both national classification and IPC

**B.    FIELDS SEARCHED**

Minimum documentation searched (classification system followed by classification symbols)

A61K A61P

Documentation searched other than minimum documentation to the extent that such documents are included in the fields searched

Electronic data base consulted during the international search (name of data base and, where practicable, search terms used)

CNTXT; ENTXTC; VCN; CJFD; DWPI; CNKI; WFNPL; NPTXT; NPABS; Web of Science; STNext; 读秀, DUXIU: 徐宇虹, 脂质体, 抗体, 化合价, 数量, 个数, her2, CD3, xu hongyu, valence, antibody, liposomes, number, quantity

**C.    DOCUMENTS CONSIDERED TO BE RELEVANT**

| Category* | Citation of document, with indication, where appropriate, of the relevant passages | Relevant to claim No. |
|---|---|---|
| PX | LI, Huimin et al. "Surface Ligand Valency and Immunoliposome Binding: when More Is Not Always Better" *Pharmaceutical Research*, Vol. 38, 31 August 2021 (2021-08-31), paragraphs 1593-1600 | 1-16 |
| X | CAO, Jing et al. "Selective Targeting and Eradication of LGR5+ Cancer Stem Cells Using RSPO-Conjugated Doxorubicin Liposomes" *Molecular Cancer Therapeutics*, Vol. 17, No. 7, 31 July 2018 (2018-07-31), pp. 1475-1485 | 1-4, 8-16 |
| X | CN 107252417 A (MERRIMACK PHARMACEUTICALS INC.) 17 October 2017 (2017-10-17) claims 1-15 | 1-4, 8-16 |
| Y | CAO, Jing et al. "Selective Targeting and Eradication of LGR5+ Cancer Stem Cells Using RSPO-Conjugated Doxorubicin Liposomes" *Molecular Cancer Therapeutics*, Vol. 17, No. 7, 31 July 2018 (2018-07-31), pp. 1475-1485 | 5-7 |
| Y | CN 107252417 A (MERRIMACK PHARMACEUTICALS INC.) 17 October 2017 (2017-10-17) claims 1-15 | 5-7 |

☑ Further documents are listed in the continuation of Box C.          ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **06 July 2022** | **09 August 2022** |

| Name and mailing address of the ISA/CN | Authorized officer |
|---|---|
| **China National Intellectual Property Administration (ISA/ CN)** **No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

**INTERNATIONAL SEARCH REPORT**

| International application No. |
|---|
| **PCT/CN2022/094222** |

**C.    DOCUMENTS CONSIDERED TO BE RELEVANT**

| Category* | Citation of document, with indication, where appropriate, of the relevant passages | Relevant to claim No. |
|---|---|---|
| Y | WO 2019052401 A1 (SHANDONG JIAOTONG UNIVERSITY) 21 March 2019 (2019-03-21)<br>description, p. 2, paragraph 5, and p. 3, paragraphs 2 and 6 | 5-7 |

Form PCT/ISA/210 (second sheet) (January 2015)

<table>
<tr><td>**INTERNATIONAL SEARCH REPORT**</td><td>International application No.<br>**PCT/CN2022/094222**</td></tr>
</table>

| Box No. I | Nucleotide and/or amino acid sequence(s) (Continuation of item 1.c of the first sheet) |
|---|---|

1. With regard to any nucleotide and/or amino acid sequence disclosed in the international application, the international search was carried out on the basis of a sequence listing:

    a. ☑ forming part of the international application as filed:

        ☑ in the form of an Annex C/ST.25 text file.

        ☐ on paper or in the form of an image file.

    b. ☐ furnished together with the international application under PCT Rule 13*ter*.1(a) for the purposes of international search only in the form of an Annex C/ST.25 text file.

    c. ☐ furnished subsequent to the international filing date for the purposes of international search only:

        ☐ in the form of an Annex C/ST.25 text file (Rule 13*ter*.1(a)).

        ☐ on paper or in the form of an image file (Rule 13*ter*.1(b) and Administrative Instructions, Section 713).

2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3. Additional comments:

Form PCT/ISA/210 (continuation of first sheet) (January 2015)

<table>
<tr><td><b>INTERNATIONAL SEARCH REPORT</b></td><td>International application No.<br><br>**PCT/CN2022/094222**</td></tr>
</table>

| Box No. II | Observations where certain claims were found unsearchable (Continuation of item 2 of first sheet) |

This international search report has not been established in respect of certain claims under Article 17(2)(a) for the following reasons:

1. ☑ Claims Nos.: **17**
because they relate to subject matter not required to be searched by this Authority, namely:

    [1]   The method set forth in claim 17 is a method for preventing and treating diseases, which is implemented on a living human or animal body and directly aimed at the prevention and/or treatment of diseases, belongs to methods for the treatment of a human or animal body, and falls within the cases listed in PCT Rule 39.1(iv) for which no international search is required.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (January 2015)

**INTERNATIONAL SEARCH REPORT**
Information on patent family members

International application No.

**PCT/CN2022/094222**

| Patent document cited in search report | | | Publication date (day/month/year) | Patent family member(s) | | | Publication date (day/month/year) |
|---|---|---|---|---|---|---|---|
| CN | 107252417 | A | 17 October 2017 | IL | 226800 | D0 | 31 July 2013 |
| | | | | US | 2016038417 | A1 | 11 February 2016 |
| | | | | CN | 103327983 | A | 25 September 2013 |
| | | | | KR | 20140041396 | A | 04 April 2014 |
| | | | | MX | 318072 | B | 17 February 2014 |
| | | | | US | 2017189335 | A1 | 06 July 2017 |
| | | | | JP | 2013544851 | A | 19 December 2013 |
| | | | | US | 2014023698 | A1 | 23 January 2014 |
| | | | | US | 2016038416 | A1 | 11 February 2016 |
| | | | | AU | 2017201983 | A1 | 01 June 2017 |
| | | | | MX | 2013006430 | A | 09 July 2014 |
| | | | | AU | 2011338487 | A1 | 18 July 2013 |
| | | | | CA | 2820245 | A1 | 14 June 2012 |
| | | | | JP | 2017025087 | A | 02 February 2017 |
| | | | | WO | 2012078695 | A2 | 14 June 2012 |
| | | | | EP | 2648738 | A2 | 16 October 2013 |
| WO | 2019052401 | A1 | 21 March 2019 | EP | 3689336 | A1 | 05 August 2020 |
| | | | | JP | 2020533360 | A | 19 November 2020 |
| | | | | CN | 109498817 | A | 22 March 2019 |
| | | | | JP | 2022087135 | A | 09 June 2022 |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- Optimization of drug combinations using Feedback System Control. *Nature Protocols.,* 2016, vol. 11, 302-315